# EUROPEAN PATENT SPECIFICATION

(11) **EP 4 342 880 B1**
(45) Date of publication and mention of the grant of the patent: **10.09.2025**
(21) Application number: 22836593.8
(22) Date of filing: 10.05.2022
(51) Int. Cl.: C07C 219/06, C07C 219/16, A61K 39/12, A61K 9/127, A61K 9/00, A61P 31/14, A61K 39/00, A61P 31/00, A61P 35/00, A61K 9/50, A61K 47/18, A61K 31/713, C07C 215/14, C07C 233/36, C07C 229/12, C07C 255/24, A61K 9/1272, A61K 31/7105

(54) **IONIZABLE LIPID COMPOUND FOR NUCLEIC ACID DELIVERY AND LNP COMPOSITION THEREOF**
IONISIERBARE LIPIDVERBINDUNG ZUR NUKLEINSÄUREABGABE UND LNP-ZUSAMMENSETZUNG DAVON
COMPOSÉ LIPIDIQUE IONISABLE POUR L'ADMINISTRATION D'ACIDE NUCLÉIQUE ET COMPOSITION LNP DE CELUI-CI

(30) Priority: 07.07.2021 CN 202110770325; 30.01.2022 CN 202210114477
(43) Date of publication of application: 27.03.2024
(73) Proprietor: Jenkem Technology Co., Ltd. (Tianjin), Tianjin 300462 (CN); Cansino (Shanghai) Biological Research Co., Ltd., Shanghai 200131 (CN)
(72) Inventor: HAO, Jing, Tianjin 300462 (CN); WANG, Qingbin, Tianjin 300462 (CN); YAN, Shengyong, Tianjin 300462 (CN); WANG, Haomeng, Shanghai 200131 (CN); YAN, Zhihong, Shanghai 200131 (CN); LIU, Jian, Shanghai 200131 (CN); YU, Xuefeng, Tianjin 300457 (CN); QIU, Dongxu, Shanghai 200131 (CN); LIN, Meina, Tianjin 300462 (CN); GUO, Jun, Tianjin 300462 (CN); XIONG, Yanli, Tianjin 300462 (CN); ZHU, Tao, Tianjin 300457 (CN); ZHAO, Xuan, Tianjin 300462 (CN)
(74) Representative: HGF
(86) International application number: PCT/CN2022/091881
(87) International publication number: WO 2023/279836

(56) References cited:
- EP-A1- 3 315 125
- WO-A1-2009/122220
- WO-A1-2018/232120
- WO-A1-2020/252589
- WO-A1-2021/000041
- WO-A1-2021/055835
- WO-A1-2021/055849
- WO-A1-2021/095876
- WO-A1-2022/140239
- WO-A1-2023/143591
- WO-A1-2023/143600
- WO-A1-2023/143601
- WO-A2-2017/049245
- WO-A2-2023/178065
- CN-A- 110 352 071
- CN-A- 111 356 444
- CN-A- 114 149 337
- JP-A- 2005 015 377
- US-A1- 2017 210 697
- US-A1- 2021 087 135
- LINXIAN LI ET AL: "Combinatorial Synthesis and High-Throughput Screening of Alkyl Amines for Nonviral Gene Delivery", BIOCONJUGATE CHEMISTRY, vol. 24, no. 9, 18 September 2013 (2013-09-18), US, pages 1543 - 1551, XP055262549, ISSN: 1043-1802, DOI: 10.1021/bc400158w

## Description

### TECHNICAL FIELD

The present invention belongs to the field of biomedicines, and particularly relates to a new type ionizable lipid for nucleic acid delivery and use thereof in the delivery of a bioactive substance.

### BACKGROUND

Gene therapy refers to the use of molecular biological methods to introduce a target gene into a patient's body and express it to correct or compensate for diseases caused by gene defects and abnormalities, and also refers to the introduction of nucleic acids into cells to inhibit the expression of a target gene (gene silencing) or increase the expression of a target gene (gene activation) to achieve the purpose of treating diseases. Gene therapy, as a new method for disease treatment that combines modern medicine and molecular biology, is gradually occupying an important position in the medical field. At present, the use of "expressing a certain gene" and "expressing a certain protein" for treatment is mainly achieved through the introduction of plasmid DNA and mRNA, while the use of "inhibiting a certain gene" for treatment is mainly achieved through siRNA or microRNA (miRNA) (i.e., RNAi technology).

Gene therapy approaches based on siRNA and mRNA have unique advantages not found in other types of nucleic acid drugs. However, a common difficulty in the development of mRNA and siRNA is how to deliver them efficiently into cells at the target site.

At present, the delivery systems for nucleic acids mostly use different types of lipid compounds, e.g., lipid nanoparticles (LNPs), GalNac, lipopolyplexes (LPPs), etc, for delivery. LNPs are generally prepared from four types of lipids in a certain proportion, the four types of lipids typically including a cationic lipid, a neutral lipid, a steroid lipid, and a polymer conjugated lipid. A cationic liposome is positively charged, and has electrostatic interaction with a negatively-charged membrane lipid in an endosome, the membrane lipid is transferred from outside cavity of the endosome to inside cavity, and forms a neutral electron pair with the positive charge, and a gene drug is separated from the cationic liposome and enters the nucleus.

Patent documents CN110352071A and CN1882693A disclose a cationic lipid compound and use of the lipid in the preparation of a lipid nanoparticle or a lipid mixture for delivering a bioactive substance into a body.

Patent document US20200197510A1 discloses a respiratory virus ribonucleic acid vaccine and a combination vaccine, and a method for using a vaccine and a composition comprising the vaccine.

"Li L, Wang F, Wu Y, Davidson G, Levkin PA. Combinatorial synthesis and high-throughput screening of alkyl amines for nonviral gene delivery. Bioconjugate Chemistry. 2013 Sep 18;24(9):1543-51." discloses a series of alkylamine lipidoids, while they show different performances in transfection efficiency.

Patent publications Nos. WO2018232120A1, WO2021055835A1 disclose lipid compounds and lipid nanoparticle compositions prepared from them, they have varying performances in transfection and delivery of bioactive reagents.

Although a series of cationic lipid compounds have been reported in the prior art, there is still a need to provide a lipid compound with efficient and stable delivery performance.

### SUMMARY

In a first aspect, the present disclosure provides a lipid compound having the structure of formula I below: wherein, 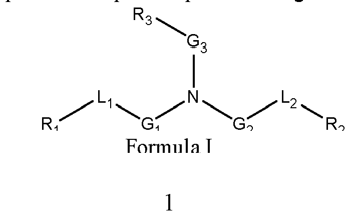
at least one of L₁ and L₂ is -O(C=O)O- or -NRa-, and
the other one of L₁ or L₂ is -O-, -O(C=O)O-, -(C=O)NRa-, -NRa(C=O)-, -NRa-, -O(C=O)-, -(C=O)O-, -C(=O)-, - S(O)x-, -S-S-, -C(=O)S-, -SC(=O)-, -NRaC(=O)NRa-, -OC(=O)NRa-, or -NRaC(=O)O-;
G₁ and G₂ are each independently unsubstituted C₁-C₁₂ alkylene or C₁-C₁₂ alkenylene;
G₃ is C₁-C₂₄ alkylene, C₁-C₂₄ alkenylene, C₃-C₈ cycloalkylene, or C₃-C₈ cycloalkenylene;
Ra is H or C₁-C₁₂ hydrocarbyl;
R₁ and R₂ are each independently C₆-C₂₄ alkyl or C₆-C₂₄ alkenyl;
R₃ is H, OH, OR₄, CN, -C(=O)OR₄, -OC(=O)R₄, or -NR₅C(=O)R₄;
R₄ is C₁-C₁₂ hydrocarbyl;
R₅ is H or C₁-C₆ hydrocarbyl;
x is 0, 1 or 2.

Furthermore, the Ra is H or C₁-C₆ alkyl (e.g., methyl, ethyl, n-propyl, or n-butyl), and especially H.

Furthermore, L₁ and L₂ in the structure of formula I are independently selected from -O(C=O)O- and -NH-.

Furthermore, L₁ and L₂ in the structure of formula I are -O(C=O)O-, or L₁ and L₂ are -NH-.

Furthermore, R₁ and R₂ in the structure of formula I each independently have the following structure: 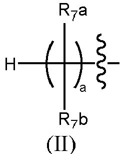
wherein, R₇ₐ and R_{7b} are independently H or C₁-C₁₂ hydrocarbyl at each occurrence;
and a is an integer of 2 to 12 (e.g., 2, 3, 4, 5, 6, 7, 8, 9, 10, 11 or 12), and preferably, a is an integer of 8 to 12;
furthermore, R₇ₐ, R_{7b} and a in the structure of formula II are each selected such that R₁ and R₂ each
independently comprise 6 to 20 carbon atoms.

Furthermore, R₇ₐ with at least one occurrence in the structure of formula II is H, and preferably, R₇ₐ is H at each occurrence.

Furthermore, R_{7b} with at least one occurrence in the structure of formula II is C₁-C₈ hydrocarbyl, and especially C₁-C₈ alkyl, e.g., methyl, ethyl, n-propyl, isopropyl, n-butyl, isobutyl, *tert*-butyl, *n*-hexyl, or *n*-octyl.

Furthermore, R₁ or R₂ or both in the structure of formula I have one of the following structures:

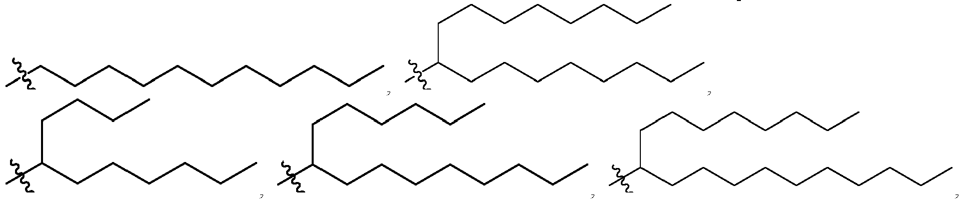

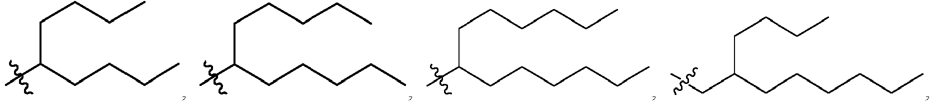

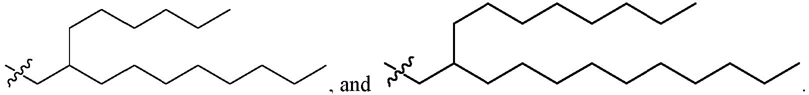

Furthermore, the lipid compound of the present disclosure has the following structure (IA):

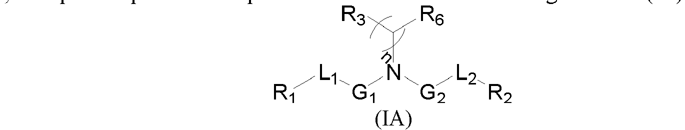

Furthermore, R₆ in the structure of formula IA is independently H, OH, or C₁-C₂₄ hydrocarbyl at each occurrence; furthermore, n in the structure of formula IA is an integer of 1 to 15 (e.g., 1, 2, 3, 4, 5, 6, 7, 8, 9, 10, 11, 12, 13, 14 or 15).

Furthermore, the lipid compound of the present disclosure has the following structure (IB): 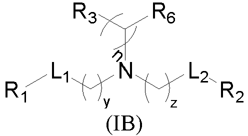 wherein y and z are each independently an integer of 1 to 12.

Furthermore, n in the structure of formula IB is an integer of 2 to 12, and preferably, n is 2, 3, 4, 5 or 6.

Furthermore, y and z in the structure of formula IB are each independently an integer of 2 to 10 (e.g., 2, 3, 4, 5, 6, 7, 8, 9 or 10), and preferably, an integer of 4 to 9.

Furthermore, R₆ in the structure of formula IA is independently H, OH, or C₁-C₆ alkyl at each occurrence (e.g., methyl, ethyl, n-propyl, or n-butyl), and especially H.

Furthermore, the R₅ is H or C₁-C₆ alkyl (e.g., methyl, ethyl, n-propyl, or n-butyl), and especially H.

Furthermore, the R₄ is C₁-C₆ alkyl, e.g., methyl, ethyl, n-propyl, isopropyl, or n-butyl.

Furthermore, the R₃ is selected from: H, OH, OCH₃, OCH₂CH₃, CN, -C(=O)OCH₃, -C(=O)OCH₂CH₃, - OC(=O)CH₃, -OC(=O)CH₂CH₃, -NHC(=O)CH₃, and -NHC(=O)CH₂CH₃.

The present invention else provides a lipid compound having the following structure 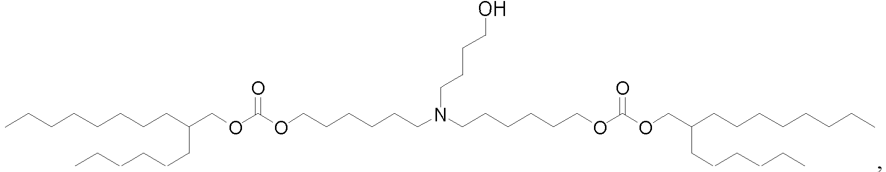 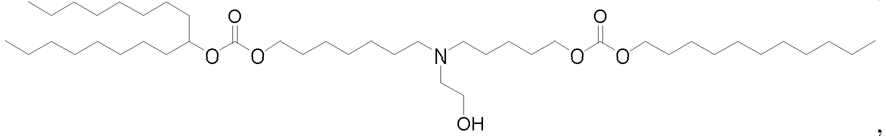 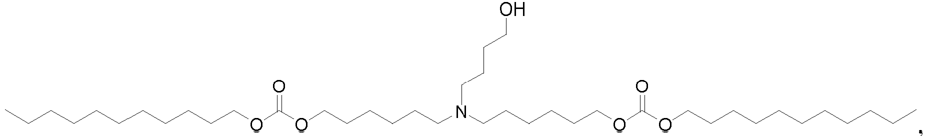 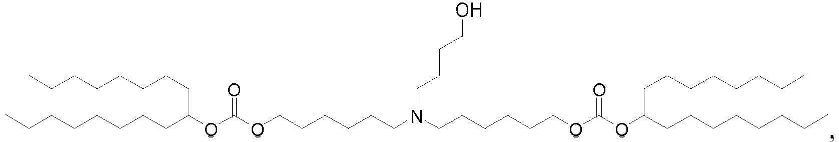 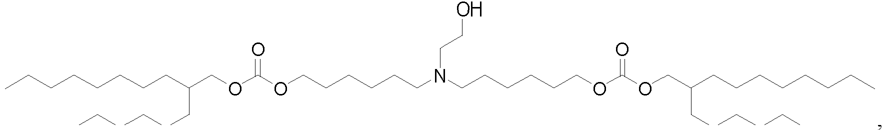 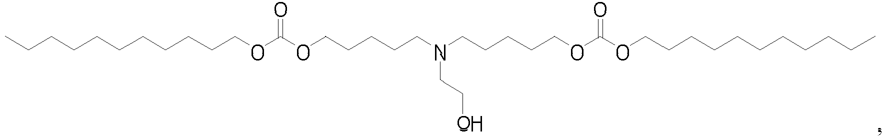 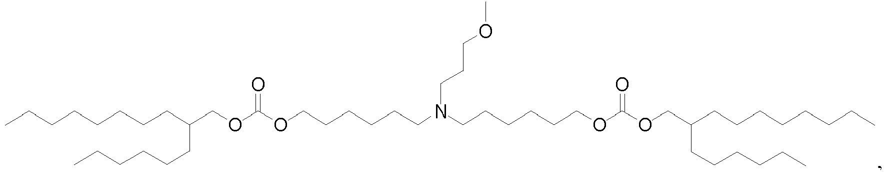 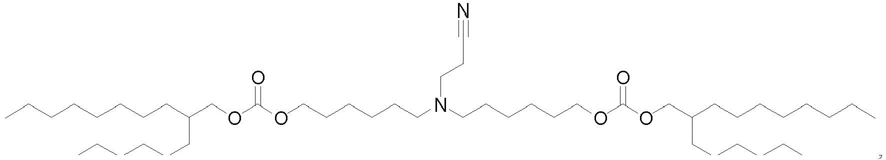 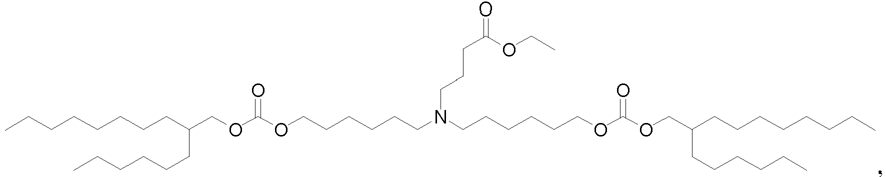 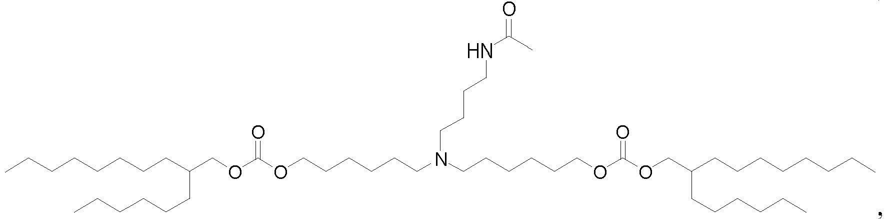 or

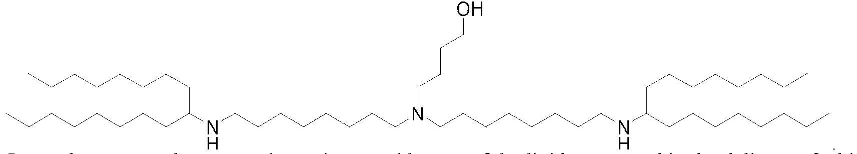

In yet another aspect, the present invention provides use of the lipid compound in the preparation of a bioactive substance delivery system.

Furthermore, the bioactive substance may be a small molecular compound, a nucleic acid, an oligopeptide, etc.

Preferably, the bioactive substance is a nucleic acid.

Furthermore, the bioactive substance is DNA or RNA.

Furthermore, the DNA includes a non-coding DNA (an antisense DNA) or a coding DNA.

Furthermore, the RNA includes an antisense RNA, a saRNA, a mRNA, a lncRNA, a miRNA, a siRNA, a piRNA, a gRNA, a tsRNA, etc.

Furthermore, the nucleic acid is used for preventing and/or treating cancer, inflammation, fibrotic disease, autoimmune disease, infection, psychiatric disorder, hematological disorder, chromosomal disease, genetic disease, connective tissue disease, digestive disease, ear-nose-throat disease, endocrine disease, eye disease, reproductive disease, heart disease, kidney disease, lung disease, metabolic disorder, oral disease, musculoskeletal disease, neonatal screening, nutritional disease, parasitic disease, skin disease, etc.

Furthermore, the present invention also provides use of the lipid compound in the delivery of an siRNA to a cell or an organ.

Furthermore, the present invention also provides use of the lipid compound in the preparation of a lipid or lipid nanoparticle delivery system.

Furthermore, the present invention also provides use of the lipid compound in the delivery of an mRNA vaccine to a cell or an organ.

Furthermore, the present invention also provides use of the lipid compound in the preparation of an mRNA vaccine. Preferably, the vaccine may be used for preventing cancer, virus infection, bacterial infection, fungal infection, etc. The viruses include, but are not limited to: norovirus, Rbola virus, coronavirus (including novel coronavirus SARS-CoV-2), cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, measles virus, etc. In one embodiment of the present invention, the mRNA vaccine is a SARS-CoV-2 mRNA vaccine

In still another aspect, the present invention provides a lipid composition comprising a bioactive substance and the lipid compound of the present invention.

Furthermore, the lipid composition may be prepared by a conventional method in the art, e.g., a heating method, a reverse evaporation method, or a mixing method.

Furthermore, the heating method comprises adding an organic solvent solution of a lipid compound to an aqueous solution of a bioactive substance to obtain a mixed solution, and heating the mixed solution at an appropriate temperature. Preferably, the heating temperature is 25 °C-100 °C. Preferably, the heating time is 10 minutes-24 hours.

Furthermore, the reverse evaporation method comprises mixing an aqueous solution of a bioactive substance with an organic solvent solution of a lipid compound to obtain a mixed solution.

In still another aspect, the present invention provides a lipid nanoparticle comprising a bioactive substance and the lipid compound of the present invention.

Furthermore, the lipid nanoparticle further comprises a polyethylene glycol lipid, a steroidal lipid, and a neutral lipid.

Furthermore, the polyethylene glycol lipid is selected from 2-[(polyethylene glycol)-2000]-N,N-ditetradecylacetamide (ALC-0159), 1,2-dimyristoyl-sn-glyceromethoxypolyethylene glycol (PEG-DMG), 1,2-distearyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE) or PEG-1,2-dimyristoylacyloxypropyl-3-amine (PEG-c-DMA).

Furthermore, the neutral lipid is selected from: 1,2-distearoyl-sn-glycerol-3-phosphocholine (DSPC), 1,2-dipalmitoyl-sn-glycerol-3-phosphocholine (DPPC), 1,2-dioleoyl-sn-glycerol-3-phosphoethanolamine (DOPE), 1,2-dipalmitoyl-sn-glycerol-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), or 1-palmitoyl-2-oleoyl phosphatidylethanolamine (POPE).

Furthermore, the steroid lipid is selected from: avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, campesterol, epicholesterol, ergosterol, fucosterol, hexahydrosterol, hydroxycholesterol, lanosterol, photosterol, fucosterol, sitostanol, sitosterol, stigmastanol, stigmasterol, cholic acid, glycocholic acid, taurocholic acid, deoxycholic acid, and lithocholic acid.

Furthermore, in the lipid nanoparticle of the present invention, the polyethylene glycol lipid is ALC-0159, and/or the steroid lipid is cholesterol, and/or the neutral lipid is DSPC.

Furthermore, in the lipid nanoparticle of the present invention, the polyethylene glycol lipid is DMG-PEG2000, and/or the steroid lipid is cholesterol, and/or the neutral lipid is DSPC.

In one embodiment of the present invention, in the lipid nanoparticle, the lipid compound of the present invention is 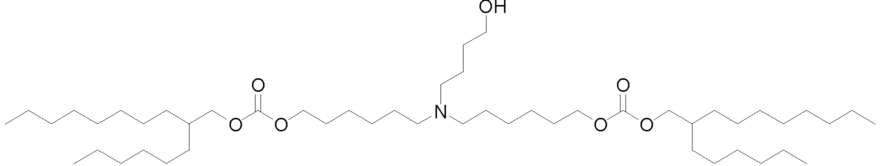 or

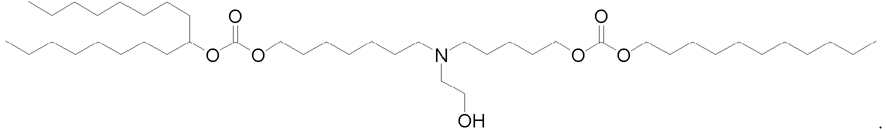

Furthermore, in the lipid nanoparticle of the present invention, the molar ratio of the lipid compound, the neutral lipid, the steroid lipid, and the polyethylene glycol lipid of the present invention is (40-60):(5-20):(30-50):(0.5-5), and preferably (45-55):(8-12):(35-45):(1-2); in one embodiment of the present invention, the molar ratio is 49:10:39.5:1.5.

The lipid nanoparticle of the present invention may be prepared by using a conventional method for preparing a lipid nanoparticle in the art, e.g., high pressure homogenization, emulsion precipitation, ultrasonic dispersion, etc. In still another aspect, the present invention provides a pharmaceutical composition, wherein the pharmaceutical composition comprises the lipid composition of the present invention or the lipid nanoparticle of the present invention, and a pharmaceutically acceptable excipient.

The pharmaceutically acceptable excipient of the present invention is, for example, a carrier, an adjuvant, a diluent, etc.

The lipid composition, the lipid nanoparticle or the pharmaceutical composition of the present invention may deliver the bioactive substance by oral administration, inhalation or injection.

In yet another aspect, the present invention provides an ionizable lipid compound as defined in the claims used in a method for delivering a bioactive substance, which comprises administering to a human in need thereof the lipid mixture of the present invention, the lipid nanoparticle of the present invention or the pharmaceutical composition of the present invention.

The lipid compound of the present invention is used for preparing a PEG-lipid/cationic lipid/neutral lipid/steroid lipid-mRNA nanoparticle (LNP), which shows that the mRNA LNP by using the lipid compound of the present invention as a cationic lipid has better stability and transfection efficiency, and can cause a higher specific antibody response in an experimental animal body.

### BRIEF DESCRIPTION OF THE DRAWINGS

FIG. 1 shows a Zeta potential detection spectrum of sample 1.
FIG. 2 shows a Zeta potential detection spectrum of sample 2.
FIG. 3 shows a Zeta potential detection spectrum of sample 3.
FIG. 4 shows a Zeta potential detection spectrum of sample 4.
FIG. 5 shows a Zeta potential detection spectrum of sample 5.
FIG. 6 shows a Zeta potential detection spectrum of sample 6.
FIG. 7 shows a Zeta potential detection spectrum of sample 7.
FIG. 8 shows a Zeta potential detection spectrum of sample 8.
FIG. 9 shows a Zeta potential detection spectrum of sample 9.
FIG. 10 shows a Zeta potential detection spectrum of sample 10.
FIG. 11 shows a Zeta potential detection spectrum of sample 11.
FIG. 12 shows a Zeta potential detection spectrum of sample 12.
FIG. 13 shows an average particle size detection spectrum of sample 1.
FIG. 14 shows an average particle size detection spectrum of sample 2.
FIG. 15 shows an average particle size detection spectrum of sample 3.
FIG. 16 shows an average particle size detection spectrum of sample 4.
FIG. 17 shows an average particle size detection spectrum of sample 5.
FIG. 18 shows an average particle size detection spectrum of sample 6.
FIG. 19 shows an average particle size detection spectrum of sample 7.
FIG. 20 shows an average particle size detection spectrum of sample 8.
FIG. 21 shows an average particle size detection spectrum of sample 9.
FIG. 22 shows an average particle size detection spectrum of sample 10.
FIG. 23 shows an average particle size detection spectrum of sample 11.
FIG. 24 shows an average particle size detection spectrum of sample 12.
FIG. 25 shows the encapsulation efficiency of LNP-mRNA prepared from different cationic lipids.
FIG. 26 shows the average particle size of LNP-mRNA prepared from different cationic lipids.
FIG. 27 shows the PDI of LNP-mRNA prepared from different cationic lipids.
FIG. 28 shows the *in-vitro* expression results of LNP-mRNA prepared from different cationic lipids.
FIG. 29 shows S protein specific antibody titers (LOG values) after 14 days of primary immunization. Data significance is analyzed by multiple comparisons using a graph-based approach.
FIG. 30 shows S protein specific antibody titers (LOG values) after 14 days of secondary immunization. Data significance is analyzed by multiple comparisons using a graph-based approach.
FIG. 31 shows the inhibition rates of 4 sets of mixed samples diluted with different multiples detected by an original strain RBD protein after 14 days of secondary immunization. The protein for ELISA assay plate is ACE2-Fc, while the competitive protein is RBD protein of the original strain.
FIG. 32 shows the inhibition rates of 4 sets of mixed samples diluted with different multiples detected by Beta variant RBD protein after 14 days of secondary immunization. The protein for ELISA assay plate is ACE2-Fc, while the competitive protein is RBD protein of the Beta variant.
FIG. 33 shows intracellular cytokine staining (ICS) method for detecting specific CD8+ T cell immune response. N is a negative control group. ns means that no significant difference is found after multiple comparison analysis using a basis method, and **** means that p value is less than 0.0001.
FIG. 34 shows the detection of specific CD4+ T cell immune response using ICS method. N is a negative control group. ns means that no significant difference is found after multiple comparison analysis using a basis method.

### DETAILED DESCRIPTION

Technical solutions in the embodiments of the present invention will be described clearly and completely below with reference to the drawings. It is apparent that the described embodiments are only a part of the embodiments of the present invention, but not all of them. Based on the examples of the present invention, all other examples obtained by those of ordinary skill in the art without creative work shall fall within the protection scope of the present invention , as long as they fall within the claims.

The term "nucleic acid" described herein refers to a polymer containing at least two deoxyribonucleotides or ribonucleotides in either single- or double-stranded form and includes DNA, RNA, and hybrids thereof.

The term "lipid" described herein refers to a group of organic compounds that include, but are not limited to, esters of fatty acids and are generally characterized by being poorly soluble in water, but soluble in many organic solvents.

The term "cationic lipid" described herein refers to a lipid molecule capable of being positively charged.

The term "neutral lipid" described herein refers to an uncharged, non-phosphoglyceride lipid molecule.

The term "polyethylene glycol lipid" described herein refers to a molecule comprising a lipid portion and a polyethylene glycol portion.

The term "lipid nanoparticle" described herein refers to a particle having at least one nanoscale dimension, which comprises at least one lipid.

The term "vaccine" described herein refers to a composition suitable for application to an animal (including a human), which induces an immune response after administration with a strength sufficient to help prevent, improve, or cure clinical diseases caused by microbial infections as a minimum.

The term "delivery system" described herein refers to a formulation or composition that regulates the spatial, temporal and dose distribution of a biologically active ingredient in an organism.

### Example 1: Synthesis of Compound 1

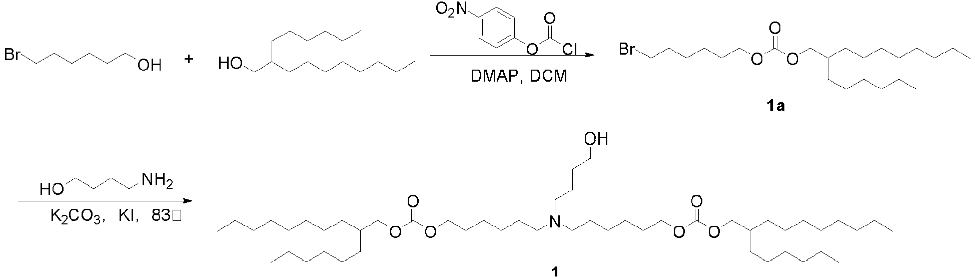

### Synthesis of 6-bromohexyl(2-hexyldecyl)carbonate (1a)

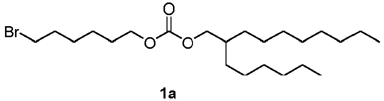

6-bromohexanol (0.91 g, 5.0 mmol) was dissolved in 30 mL of dichloromethane, 4-dimethylaminopyridine (0.90 g, 7.5 mmol) was added, and 4-nitrophenyl chloroformate (1.20 g, 6.0 mmol) was further added in portions. The resulting mixture was stirred and reacted at room temperature for 3 h, 2-hexyldecanol (1.36 g, 5.6 mmol) was added to the reaction solution, and the mixture was stirred at room temperature overnight. After the reaction was completed as detected by TLC, 20 mL of dichloromethane was added for dilution, and then the reaction solution was washed with 30 mL of saturated brine. The organic phase was dried over anhydrous sodium sulfate, filtered and concentrated, and the residue was separated by column chromatography to give 6-bromohexyl (2-hexyldecyl) carbonate **1a** (1.53 g, light yellow oil) in a yield of 68%.

MS m/z (ESI): 449.3 [M+1].

### Synthesis of compound 1

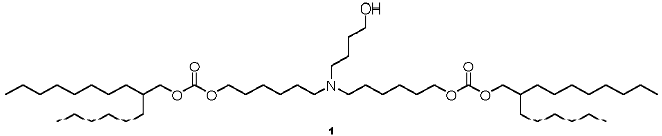

6-bromohexyl(2-hexyldecyl)carbonate (1.12 g, 2.5 mmol) was dissolved in tetrahydrofuran, then acetonitrile, 4-amino-1-butanol (89.2 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product 1 (454 mg, light yellow oil) in a yield of 55%.

MS m/z (ESI): 826.9 [M+1].

¹H NMR (300 MHz, CDCl₃): δ 4.13 (t, 4H, J = 6.6Hz), 4.05 (d, 4H, J = 5.7Hz), 3.56-3.55 (m, 2H), 2.47-2.42 (m, 6H), 1.72-1.67 (m, 10H), 1.53-1.48 (m, 8H), 1.45-1.28 (m, 52H), 0.69 (t, 12H, J = 6.2 Hz).

### Example 2: Synthesis of Compound 2

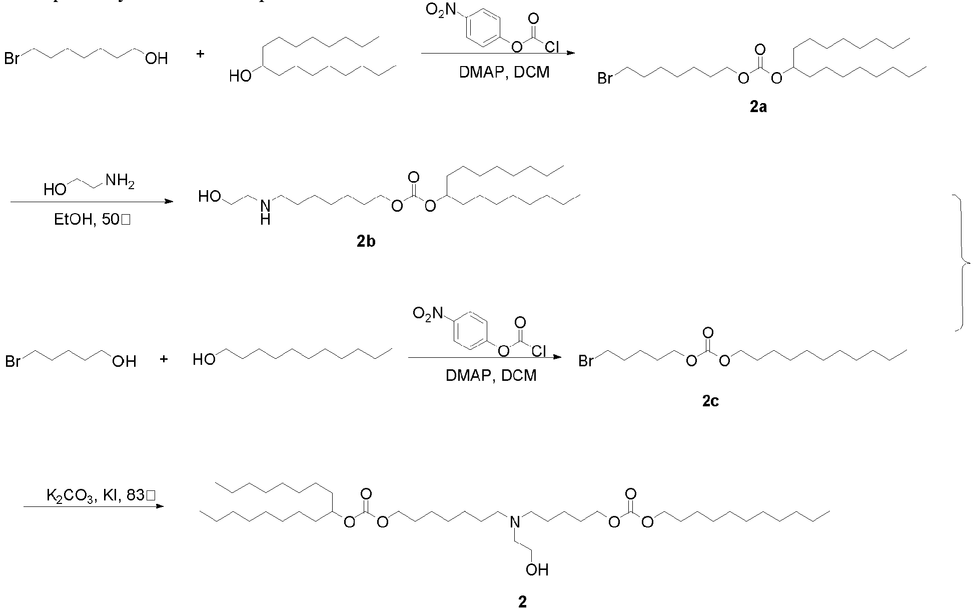

### Synthesis of 7-bromoheptylheptadecane-9-ylcarbonate (2a)

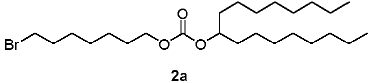

7-bromoheptanol (0.98 g, 5.0 mmol) was dissolved in 30 mL of dichloromethane, 4-dimethylaminopyridine (1.22 g, 10 mmol) was added, and then 4-nitrophenyl chloroformate (1.11 g, 5.5 mmol) was further added in portions. The resulting mixture was stirred and reacted at room temperature for 3 h, 9-hydroxyheptadecanol (1.44 g, 5.6 mmol) was added to the reaction solution, and the mixture was stirred at room temperature overnight. After the reaction was completed as detected by TLC, 20 mL of dichloromethane was added for dilution, and then the reaction solution was washed with 30 mL of saturated brine. The organic phase was dried over anhydrous sodium sulfate, filtered and concentrated, and the residue was separated by column chromatography to give 7-bromoheptylheptadecane-9-ylcarbonate 2a (1.50 g, light yellow oil) in a yield of 65%.

MS m/z (ESI): 477.3 [M+1].

### Synthesis of heptadecan-9-yl(7-((2-hydroxyethyl)amino)heptyl)carbonate (2b)

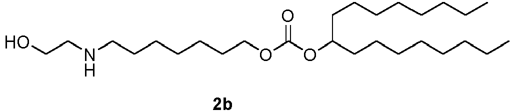

7-bromoheptylheptadecane-9-ylcarbonate (2a) (1.38 g, 3 mmol) was dissolved in 20 mL of ethanol at room temperature, ethanolamine (2.75 g, 45 mmol) was added, and the mixture was heated to 50 °C and stirred for 8 h. After the starting materials were consumed completely as monitored, the reaction solution was cooled to 45 °C, ethanol was removed by rotary drying, and the crude product was dissolved in dichloromethane, and washed with saturated brine for three times. The organic phase was dried over anhydrous sodium sulfate, and concentrated to give the product heptadecan-9-yl(7-((2-hydroxyethyl)amino)heptyl)carbonate (2b) (1.35 g, light yellow oil).

MS m/z (ESI): 458.4 [M+1].

### Synthesis of 5-bromopentylundecyl carbonate (2c)

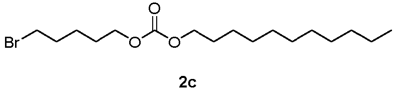

5-bromopentanol (0.84 g, 5.0 mmol) was dissolved in 30 mL of dichloromethane, 4-dimethylaminopyridine (1.22 g, 10 mmol) was added, and 4-nitrophenyl chloroformate (1.11 g, 5.5 mmol) was further added in portions. The mixture was stirred and reacted at room temperature for 3 h, undecanol (0.97 g, 5.6 mmol) was added to the reaction solution, and the mixture was stirred at room temperature overnight. After the reaction was completed as detected by TLC, 20 mL of dichloromethane was added for dilution, and then the reaction solution was washed with 30 mL of saturated brine. The organic phase was dried over anhydrous sodium sulfate, filtered and concentrated, and the residue was separated by column chromatography to give 5-bromopentylundecyl carbonate 2c (1.20 g, light yellow oil) in a yield of 66%.

MS m/z (ESI): 365.2 [M+1].

### Synthesis of compound 2

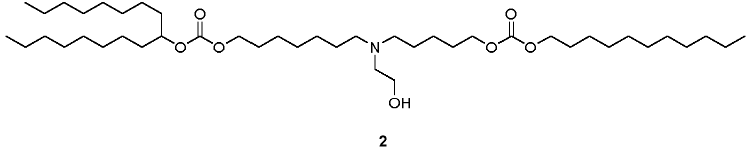

Heptadecan-9-yl(7-((2-hydroxyethyl)atnino)heptyl)carbonate (457 mg, 1.0 mmol) was dissolved in tetrahydrofuran, then acetonitrile, 5-bromopentylundecyl carbonate (437 mg, 1.2 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product 2 (440 mg, light yellow oil) in a yield of 57%.

MS m/z (ESI): 742.8 [M+1].

¹H NMR (300 MHz, CDCl₃): δ 4.71-4.68 (m, 1H), 4.15-4.10 (m, 6H), 3.53 (t, 2H, J = 5.4Hz), 2.94 (br, 1H), 2.58 (t, 2H, J = 5.4Hz), 2.45 (t, 4H, J = 5.7Hz), 1.75-1.34 (m, 62H), 0.90 (t, 9H, J = 6.3Hz).

### Example 3: Synthesis of Compound 3

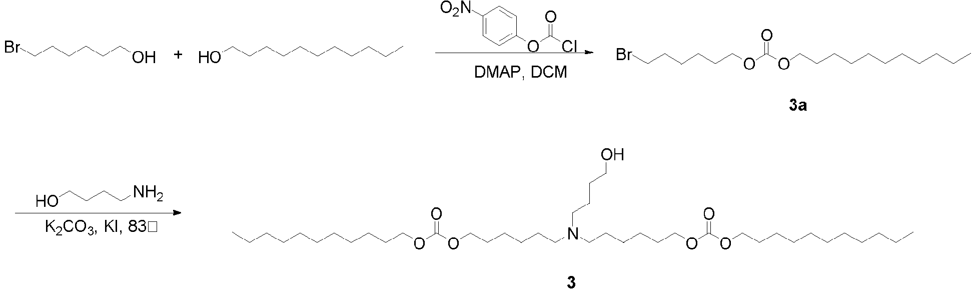

### Synthesis of 6-bromohexylundecyl carbonate (3a)

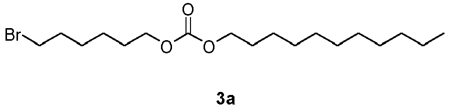

6-bromohexanol (0.91 g, 5.0 mmol) was dissolved in 30 mL of dichloromethane, 4-dimethylaminopyridine (0.90 g, 7.5 mmol) was added, and 4-nitrophenyl chloroformate (1.20 g, 6.0 mmol) was further added in portions. The mixture was stirred and reacted at room temperature for 3 h, undecanol (0.97g, 5.6 mmol) was added to the reaction solution, and the mixture was stirred at room temperature overnight. After the reaction was completed as detected by TLC, 20 mL of dichloromethane was added for dilution, and then the reaction solution was washed with 30 mL of saturated brine. The organic phase was dried over anhydrous sodium sulfate, filtered and concentrated, and the residue was separated by column chromatography to give 6-bromohexylundecyl carbonate 3a (1.25 g, light yellow oil) in a yield of 66%.

MS m/z (ESI): 379.2 [M+1].

### Synthesis of compound 3

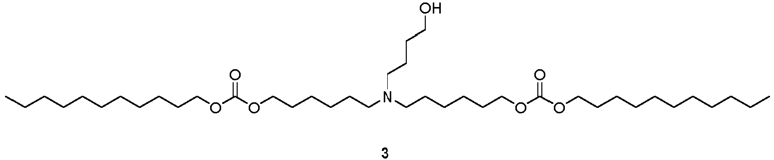

6-bromohexylundecyl carbonate (948 mg, 2.5 mmol) was dissolved in tetrahydrofuran, then acetonitrile, 4-amino-1-butanol (89.2 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product 3 (412 mg, light yellow oil) in a yield of 60%.

MS m/z (ESI): 686.8 [M+1].

¹H NMR (300 MHz, CDCl₃): δ 4.13 (t, 8H, J = 6.6Hz), 3.58 (t, 2H, J = 5.7Hz), 2.52 (t, 6H, J = 8.4Hz), 1.74-1.64 (m, 12H), 1.63-1.53 (m, 5H), 1.52-1.39 (m, 39H), 0.86 (t, 6H, J = 6.2Hz).

### Example 4: Synthesis of Compound 4

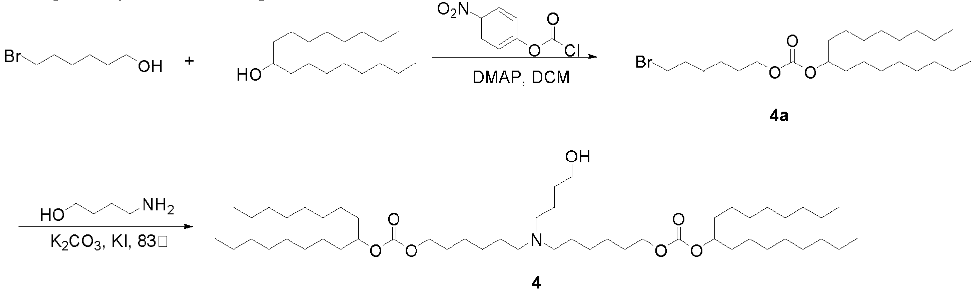

### Synthesis of 6-bromohexylheptadecan-9-ylcarbonate (4a)

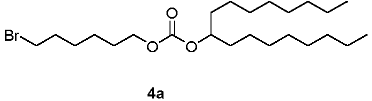

6-bromohexanol (0.91g, 5.0 mmol) was dissolved in 30 mL of dichloromethane, 4-dimethylaminopyridine (0.90g, 7.5 mmol) was added, and 4-nitrophenyl chloroformate (1.20g, 6.0 mmol) was further added in portions. The mixture was stirred and reacted at room temperature for 3 h, 9-heptadecanol (1.44 g, 5.6 mmol) was added to the reaction solution, and the mixture was stirred at room temperature overnight. After the reaction was completed as detected by TLC, 20 mL of dichloromethane was added for dilution, and then the reaction solution was washed with 30 mL of saturated brine. The organic phase was dried over anhydrous sodium sulfate, filtered and concentrated, and the residue was separated by column chromatography to give 6-bromohexylheptadecan-9-ylcarbonate **4a** (1.53 g, light yellow oil) in a yield of 66%.

MS m/z (ESI): 464.3 [M+1].

### Synthesis of compound 4

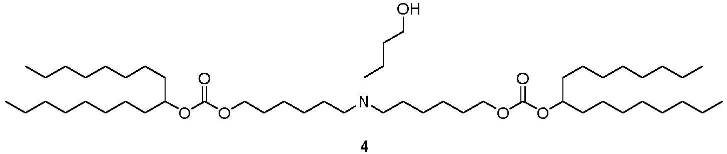

6-bromohexylheptadecan-9-ylcarbonate (1.16 g, 2.5 mmol) was dissolved in tetrahydrofuran, then acetonitrile, 4-amino-1-butanol (89.2 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **4** (502 mg, light yellow oil) in a yield of 59%.

MS m/z (ESI): 855.4 [M+1].

¹H NMR (300 MHz, CDCl₃): δ 4.71-4.68 (m, 2H), 4.13 (t, 4H, J = 6.6Hz), 3.57 (t, 2H, J = 5.4Hz), 2.49-2.44 (m, 6H), 1.74-1.28 (m, 76H), 0.90 (t, 12H, J = 6.3Hz).

### Example 5: Synthesis of Compound 5

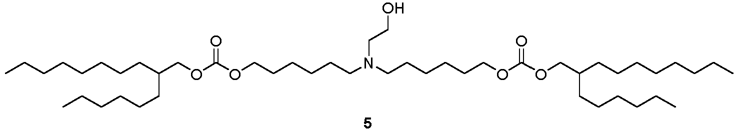

6-bromohexyl(2-hexyldecyl)carbonate (1.12 g, 2.5 mmol) was dissolved in tetrahydrofuran, then acetonitrile, ethanolamine (61.0 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **5** (487 mg, light yellow oil) in a yield of 61%.

MS m/z (ESI): 798.9 [M+1].

¹H NMR (300 MHz, CDCl₃): δ 4.14 (t, 4H, J = 6.6Hz), 4.04 (d, 4H, J = 5.7Hz), 3.54 (t, 2H, J = 5.4Hz), 2.58 (t, 2H, J = 5.4Hz), 2.46 (t, 4H, J = 7.2Hz), 1.72-1.65 (m, 6H), 1.49-1.28 (m, 61H), 0.69 (t, 12H, J = 6.2Hz).

### Example 6: Synthesis of Compound 6

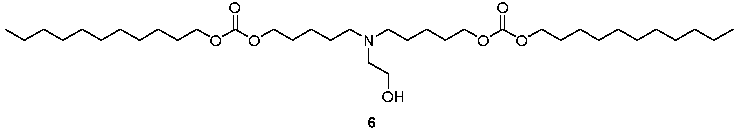

5-bromopentylundecyl carbonate (910 mg, 2.5 mmol) was dissolved in tetrahydrofuran, then acetonitrile, ethanolamine (61.0 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product 6 (410 mg, light yellow oil) in a yield of 65%.

MS m/z (ESI): 630.7 [M+1].

¹H NMR (300 MHz, CDCl₃): δ 4.10 (t, 8H, J = 6.6Hz), 3.52 (d, 2H, J = 5.4Hz), 2.83 (br, 1H), 2.57 (t, 2H, J = 5.4Hz), 2.45 (t, 4H, J = 7.2Hz), 1.73-1.62 (m, 8H), 1.52-1.39 (m, 40H), 0.69 (t, 6H, J = 6.2Hz).

### Example 7: Synthesis of Compound 7

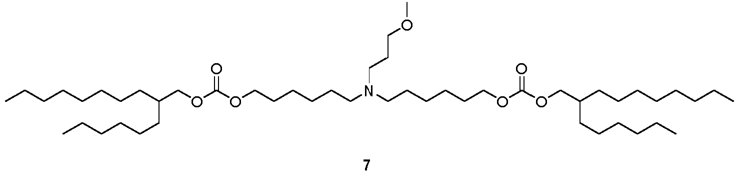

6-bromohexyl(2-hexyldecyl)carbonate (1.12 g, 2.5 mmol) was dissolved in tetrahydrofuran, then acetonitrile, 3-methoxypropylamine (89 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **7** (495 mg, light yellow oil) in a yield of 60%.

MS m/z (ESI): 826.7 [M+1].

### Example 8: Synthesis of Compound 8

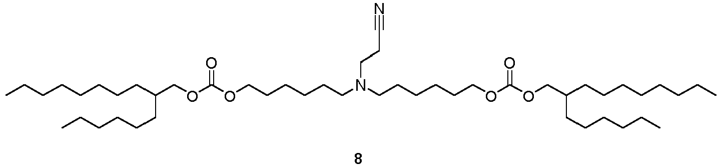

6-bromohexyl(2-hexyldecyl)carbonate (1.12 g, 2.5 mmol) was dissolved in tetrahydrofuran, then acetonitrile, 3-aminopropiononitrile (70 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **8** (469 mg, light yellow oil) in a yield of 58%.

MS m/z (ESI): 807.7 [M+1].

### Example 9: Synthesis of Compound 9

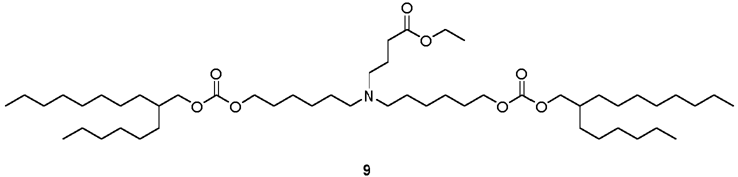

6-bromohexyl(2-hexyldecyl)carbonate (1.12 g, 2.5 mmol) was dissolved in tetrahydrofuran, then acetonitrile, ethyl 4-aminobutyrate hydrochloride (167 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **9** (546 mg, light yellow oil) in a yield of 63%.

MS m/z (ESI): 868.8 [M+1].

### Example 10: Synthesis of Compound 10

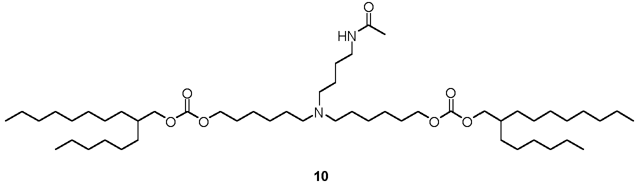

6-bromohexyl(2-hexyldecyl)carbonate (1.12 g, 2.5 mmol) was dissolved in tetrahydrofuran, then acetonitrile, *N-*(4-aminobutyl)-acetamide hydrochloride (167 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **10** (560 mg, light yellow oil) in a yield of 69%.

MS m/z (ESI): 867.8 [M+1].

### Example 11: Synthesis of Compound 11

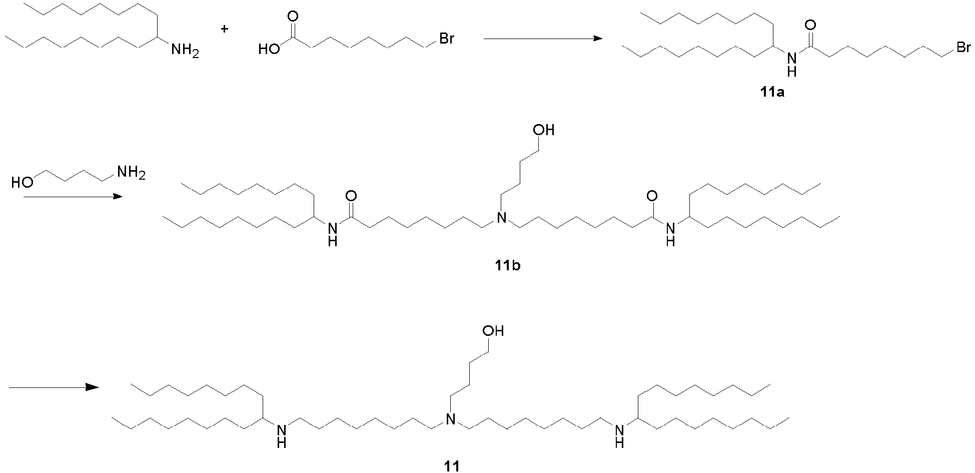

### Synthesis of 8-bromo-N-(heptadecan-9-yl)octanamide (11a)

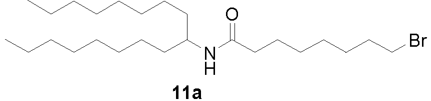

8-bromooctanoic acid (1.12 g, 5.0 mmol) was dissolved in 50 mL of dichloromethane, 1-ethyl-(3-dimethylaminopropyl)carbonyldiimine hydrochloride (1.05 g, 5.5 mmol) was added in portions at 0 °C, and 9-aminoheptadecane (1.28 g, 5.0 mmol) was added dropwise to the reaction solution after stirring for 30 min. After completion of the dropwise addition, the mixture was stirred at room temperature overnight, and after the reaction was completed as detected by TLC, the reaction solution was washed twice with 100 mL of water. The organic phase was dried over anhydrous sodium sulfate, filtered and concentrated to give compound 11a (1.95 g, yellow oil) in a yield of 82%.

MS m/z (ESI): 461.3 [M+1].

### Synthesis of compound 11b

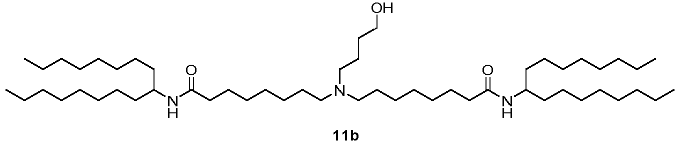

8-bromo-*N*-(heptadecan-9-yl)octanamide (1.15 g, 2.5 mmol) was dissolved in tetrahydrofuran, then acetonitrile, 4-amino-1-butanol (89.2 mg, 1.0 mmol), potassium carbonate (550 mg, 4.0 mmol), and potassium iodide (332 mg, 2.0 mmol) were added, and the resulting mixture was stirred at 83 °C for 16-20 h. The reaction solution was cooled to room temperature, and filtered, the filter residue was washed with dichloromethane, and a saturated sodium bicarbonate solution was added to the obtained filtrate, followed by extraction twice with dichloromethane. The organic phases were combined, dried over anhydrous sodium sulfate, filtered and concentrated, and the residue was separated by column chromatography to give product **11b** (534 mg, light yellow oil) in a yield of 63%.

MS m/z (ESI): 848.8 [M+1].

¹H NMR (300 MHz, CDCl₃): δ 8.10 (s, 2H), 4.21 (s, 1H), 3.46-3.4 (m, 4H), 3.02 (t, 6H, J = 6.2Hz), 2.14 (t, 4H, J = 4.8Hz), 1.57-1.47 (t, 14H, J = 6.3Hz), 1.36-1.26 (m, 66H), 0.90 (t, 12H, J = 6.3Hz).

### Synthesis of compound 11

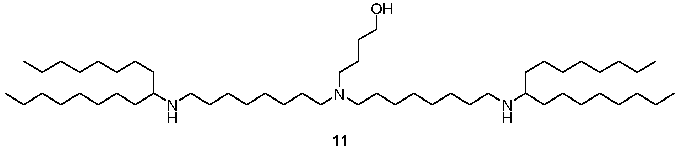

Compound 11b (1.70 g, 2 mmol) was added slowly to a solution of lithium aluminium hydride (379 mg, 10 mmol) in anhydrous tetrahydrofuran (10 mL) at 0 °C, and the mixture was heated and refluxed for 5 h. After the reaction was completed, the mixture was cooled, and then water was added to the system to make the excess reducing agent be completely decomposed. The mixture was filtered, the filter residue was washed with ethyl acetate, and the obtained filtrate was washed with water, dried over anhydrous sodium sulfate, filtered and concentrated to give compound 11 (1.45 g, yellow oil) in a yield of 90%.

MS m/z (ESI): 820.8 [M+1].

¹H NMR (300 MHz, CDCl₃): δ 4.11 (s, 1H), 3.44 (t, 2H, J = 4.8Hz), 3.32 (s, 2H), 3.00 (t, 6H, J = 6.3Hz), 2.52 (t, 4H, J = 6.3Hz), 2.48-2.43 (m, 2H), 1.61-1.56 (m, 2H), 1.36-1.26 (m, 82H), 0.86 (t, 12H, J = 4.8Hz).

### Example 12: Preparation of novel coronavirus LNP-mRNA

Novel coronavirus mRNA lipid complexes were prepared using the cationic lipids **I-II** prepared in Examples 1 and 2 of the present invention and control lipids III-IV, respectively, and the structures of the four cationic lipids are shown in the table below.

**Table 1: Cationic lipid structural formula**

| **Cationic lipid** | **Structural formula** |
|---|---|
| I | 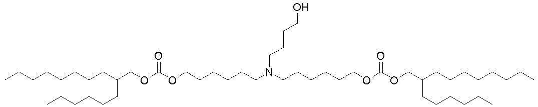 |
| II | 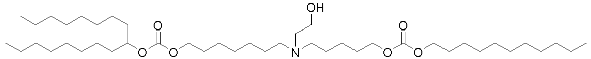 |
| III | 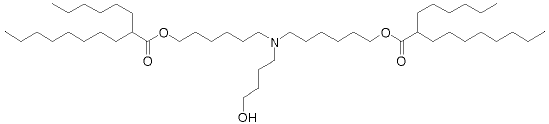 |
| IV | 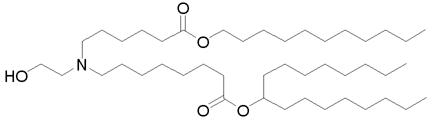 |

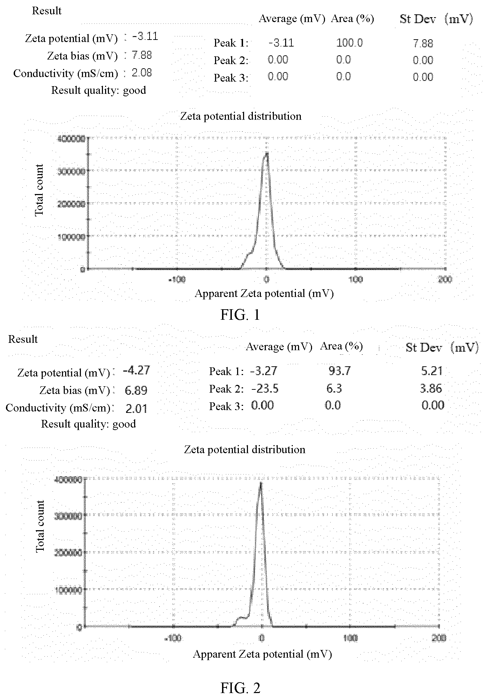

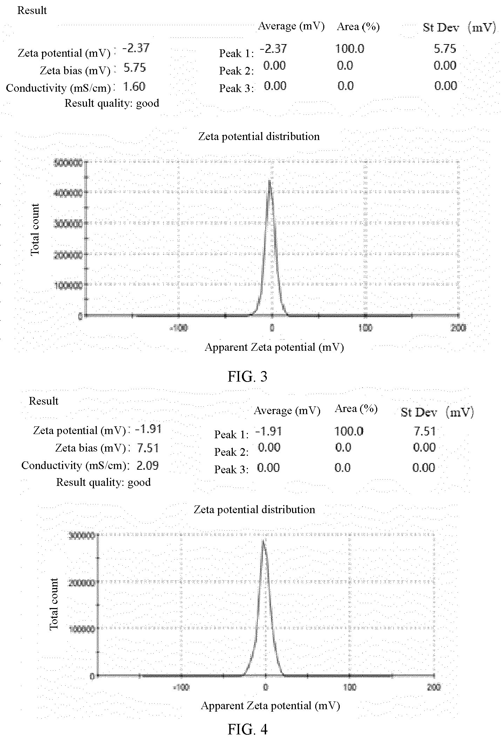

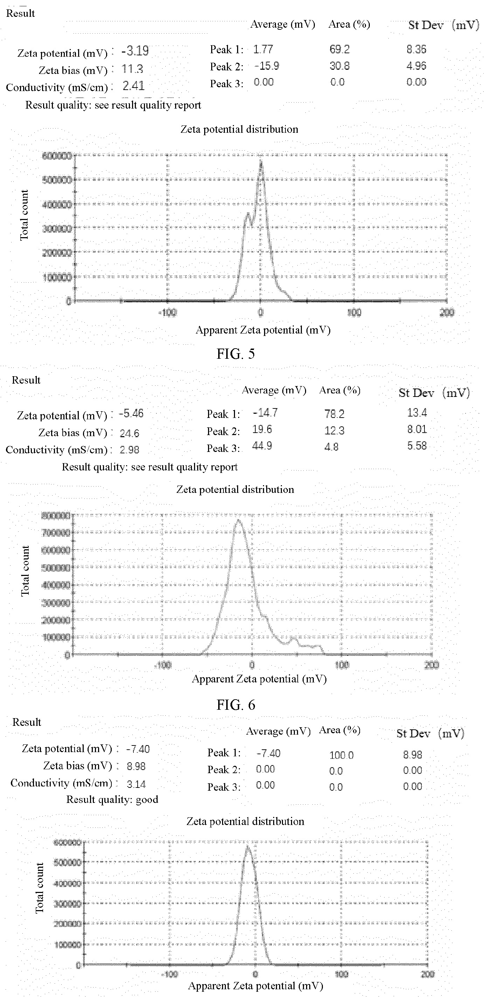

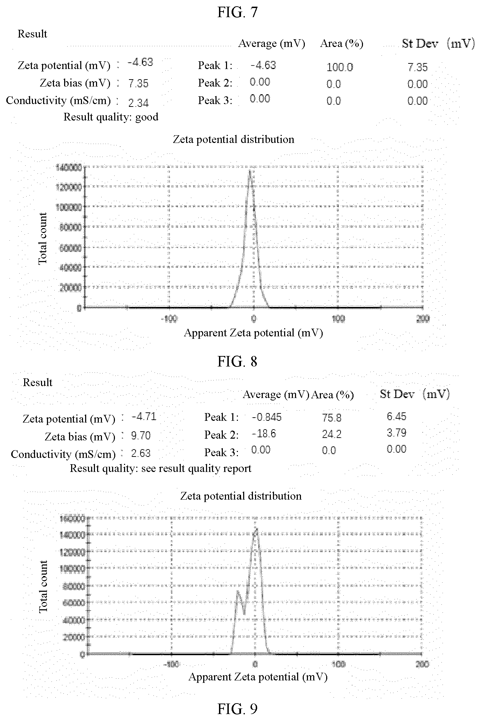

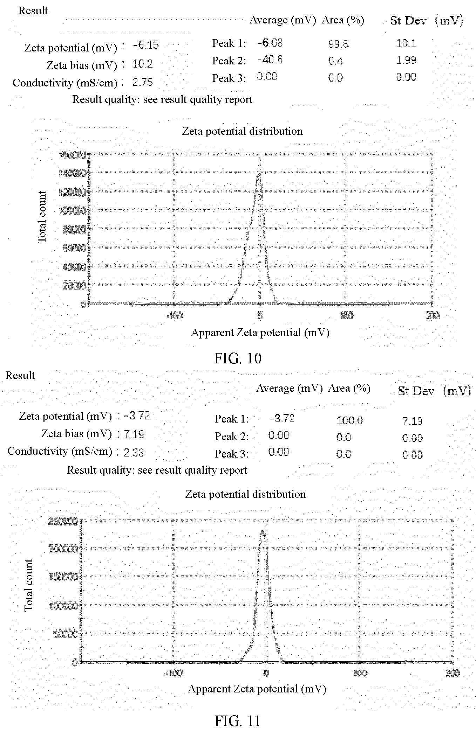

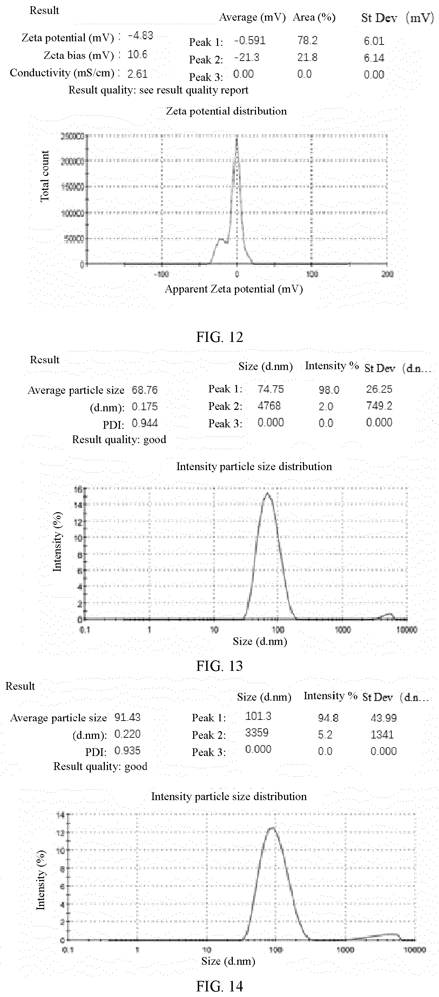

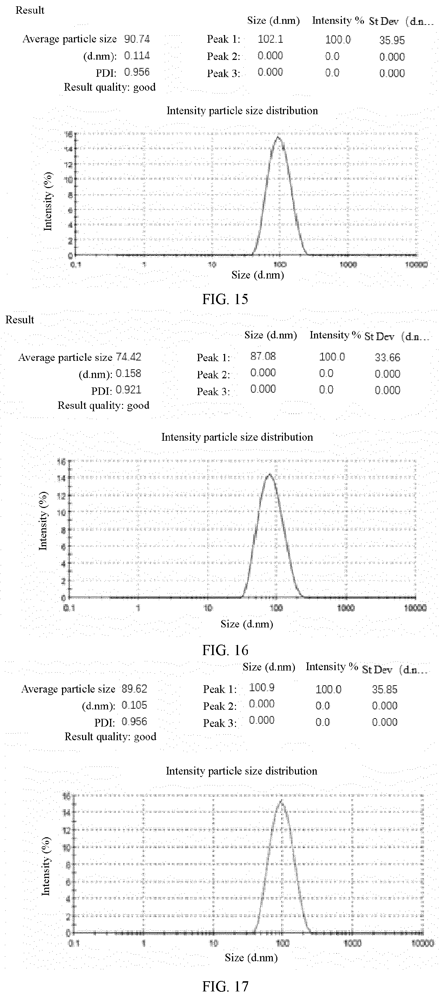

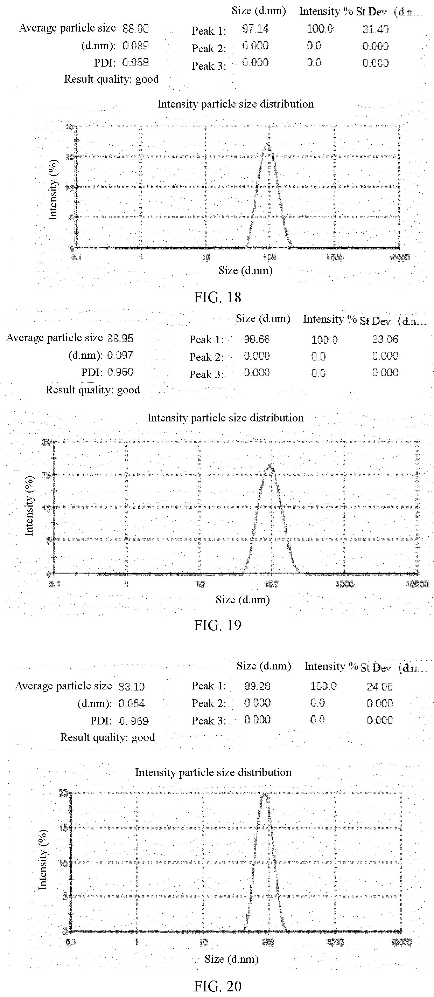

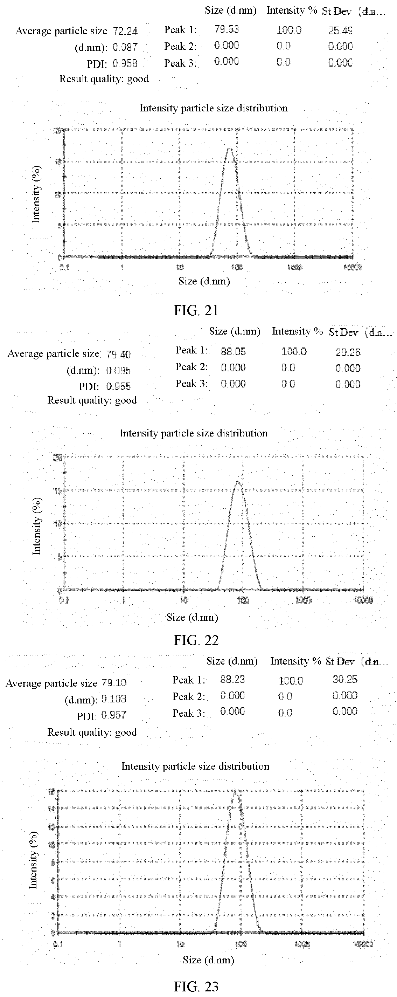

The preparation process of novel coronavirus lipid nanoparticle mRNA vaccine was as follows: mRNA stock solution was diluted to a concentration of 135 µg/mL with sodium acetate buffer, and a lipid mixed solution was prepared according to a molar ratio of cationic lipid:DSPC:cholesterol:DMG-PEG 2000 of 49:10:39.5:1.5; after the encapsulation was completed on the nano-drug manufacturing equipment, the solution was ultra-filtered and changed, and the sample was collected. The encapsulation efficiency, average particle size, PDI and Zeta potential were measured by sampling, and the results are shown in the table below and FIGs. 1-24.

**Table 2: Detection results of novel coronavirus lipid nanoparticle mRNA vaccine after different cationic lipid encapsulation**

| **Samples** | **N/P** | **Encapsulation efficiency (%)** | **Average particle size (nm)** | **PDI** | **Zeta (mV)** |
|---|---|---|---|---|---|
| 1 | 2 | 95.4 | 68.76 | 0.175 | -3.11 |
| 2 | | 96.3 | 91.43 | 0.220 | -3.27 |
| 3 | | 90.0 | 90.74 | 0.114 | -2.37 |
| 4 | | 86.1 | 72.42 | 0.158 | -1.91 |
| 5 | 4 | 96.4 | 89.62 | 0.105 | -1.77 |
| 6 | | 96.7 | 88.00 | 0.089 | -1.47 |
| 7 | | 90.2 | 88.95 | 0.097 | -7.40 |
| 8 | | 87.4 | 83.10 | 0.064 | -4.63 |
| 9 | 6 | 98.4 | 72.24 | 0.087 | -4.71 |
| 10 | | 97.5 | 79.40 | 0.095 | -6.15 |
| 11 | | 91.4 | 79.10 | 0.103 | -3.72 |
| 12 | | 90.6 | 78.48 | 0.088 | -4.83 |

Note: samples 1, 5 and 9 used the cationic lipid I, respectively; samples 2, 6 and 10 used the cationic lipid II, respectively; samples 3, 7 and 10 used the cationic lipid III, respectively; samples 4, 8 and 12 used the cationic lipid IV, respectively.

From the above results, it could be seen that under the same N/P conditions, the encapsulation efficiencies of samples 1, 2, 5, 6, 9 and 10 prepared by using the cationic lipids I and II were higher than those of samples 3, 4, 7, 8, 11 and 12 prepared by using the control cationic lipids III and IV, and thus it could be preliminarily concluded that the cationic lipids I and II had a better encapsulation effect on mRNA antigen.

### Example 13: Stability Investigation of LNP-mRNA Prepared From Different Cationic Lipids

Four LNP-mRNAs of samples 9, 10, 11 and 12 prepared in Example 12 were placed in a constant temperature incubator at 25 °C for 1, 2, 3 and 4 weeks, respectively, to investigate their stability.

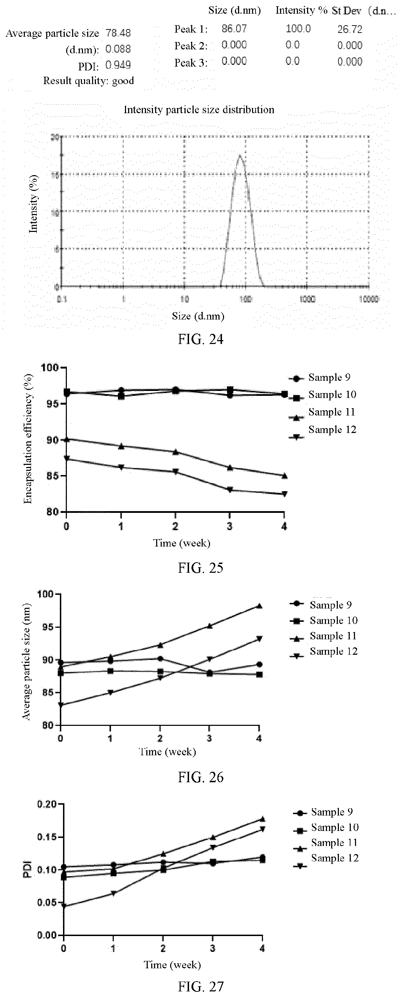

The results are shown in FIGs. 25-27, and it could be seen from the above results that the encapsulation efficiency, the average particle size and the PDI of samples 9 and 10 did not change significantly during the acceleration period, while the encapsulation efficiency of samples 11 and 12 decreased significantly and the average particle size and the PDI increased significantly during the acceleration period, and thus it could be preliminarily concluded that the stability of LNP-mRNAs prepared by the cationic lipids I and II was better than that thereof prepared by the cationic lipids III and IV.

### Example 14: In-vitro expression investigation of LNP-mRNAs prepared from different cationic lipids

Four LNP-mRNAs of samples 9, 10, 11 and 12 prepared in Example 12 were taken for detecting target gene expression of a test sample by WB method.

Cell plating: Hep3B cells were prepared, trypsinized, adjusted to a cell density of 6.5 × 10⁵ cell/mL, and seeded onto a 6-well cell culture plate at 1 mL/well, and an EMEM complete medium was supplemented to make up to 3 mL. The cells were cultured overnight in a cell incubator with 5% CO₂ at 37 °C.

Sampling: (1) RNase in the working environment was removed. The EMEM complete medium was replaced with an EMEM serum-free medium (3 mL/well). (2) The test sample was added to a 6-well cell culture plate in an amount of 25 µL/well (about 2500 ng mRNA), and meanwhile, a negative control (blank control) was set. (3) The cell culture plate was placed in a cell incubator with 5% CO₂ at 37 °C for culturing for 22-26 hours.

Cell harvesting and lysis: (1) the cells were pipetted to be completely detached from the bottom of the culture plate, the cell suspension was centrifuged at 1000 rpm for 5 minutes, and then the supernatant was discarded. (2) The cell pellet was pipetted using 150 µL of RIPA lysate and uniformly mixed, and then lysed on ice for 30 minutes, during which vortex oscillation was carried out for 4-6 times. (3) The cells after lysis were centrifuged at 15000× g for 20 min at 4 °C and the supernatant was transferred to a new EP tube and stored in a freezer at -20 °C for later use. (4) The sample from (3) was taken, the corresponding volume of 6× protein loading buffer was added, and the mixture was uniformly mixed and placed in a boiling water bath or at 100 °C for 5 minutes to be tested. Electrophoresis: a sample to be tested and protein Marker were added to a sample cell of the protein electrophoresis gel. At constant pressure, electrophoresis was stopped when a bromophenol blue indicator ran out of the gel. Membrane transfer: the protein on the protein electrophoresis gel was transferred to a nitrocellulose membrane. Blocking: the nitrocellulose membrane was placed in a blocking solution and blocked overnight at 2-8 °C or on a shaker at room temperature for 1-2 hours.

Primary antibody incubation: the nitrocellulose membrane was immersed into a certain concentration of novel coronavirus S protein specific antibody, and the mixture was incubated overnight at 2-8 °C or on a shaker at room temperature for 2 hours.

Membrane washing: the membrane was washed with PBST washing liquid for 3 times, 5 minutes per time. Primary antibody incubation: the nitrocellulose membrane was immersed into a certain concentration of horseradish peroxidase-labeled goat anti-rabbit IgG, and the mixture was incubated on a shaker at room temperature for 1 hour.

Membrane washing: the membrane was washed with PBST washing liquid for 6 times, 5 minutes per time. Imaging: a luminescent substrate was added for imaging.

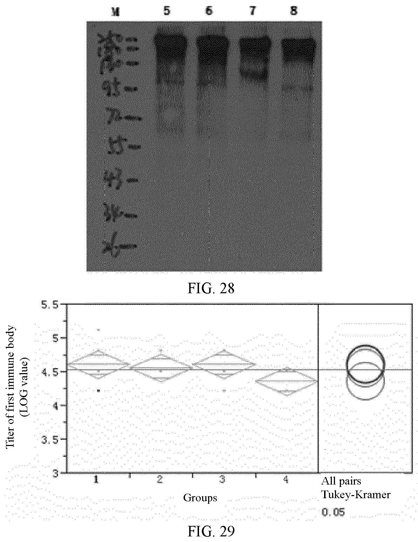

The detection results are shown in FIG. 28, wherein protein strips could be expressed *in vitro* in samples 9, 10, 11 and 12, but the protein strips of samples 9 and 10 were darker than those of samples 11 and 12, and thus it could be preliminarily concluded that the *in-vitro* transfection efficiencies of the cationic lipids I and II were better than those of the cationic lipids III and IV.

### Example 15: Mouse immunization and detection of LNP-mRNAs prepared from different cationic lipids

Female BALB/c mice aged 6-8 weeks were divided into 5 groups with 6 mice per group at random, and immunized by the intramuscular injection of hind leg. Animals in groups 1, 2, 3 and 4 were immunized with samples 9, 10, 11 and 12 (prepared in Example 12), respectively; group 5 served as a negative control group, immunized with physiological saline. Immunization was performed on day 0 and day 14, respectively, with a single immunization dose of 5 µg mRNA-LNP. Blood was collected on day 14 and day 28 of immunization and serum was isolated; mice were dissected and splenocytes were isolated after blood collection on day 28 of immunization.

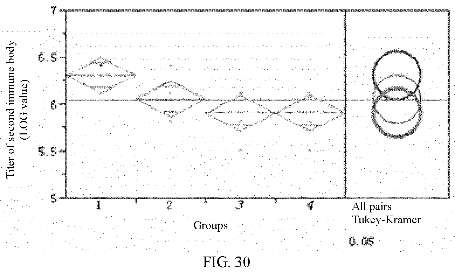

Serum samples on day 14 of immunization were used to detect the specific antibody titer against novel coronavirus S protein by ELISA. The detection results are shown in FIG. 29, where four samples all elicited higher specific antibody responses, and there was no significant difference in antibody response levels between the groups. Serum samples on day 28 of immunization were also used to detect the specific antibody titer against novel coronavirus S protein by ELISA. The detection results are shown in FIG. 30, where four samples all elicited higher specific antibody responses, wherein group 1 (sample 9) elicited the highest antibody response level, with no significant difference from group 2 (sample 10), but significantly higher than that of the positive control samples in groups 3 and 4 (samples 11 and 12).

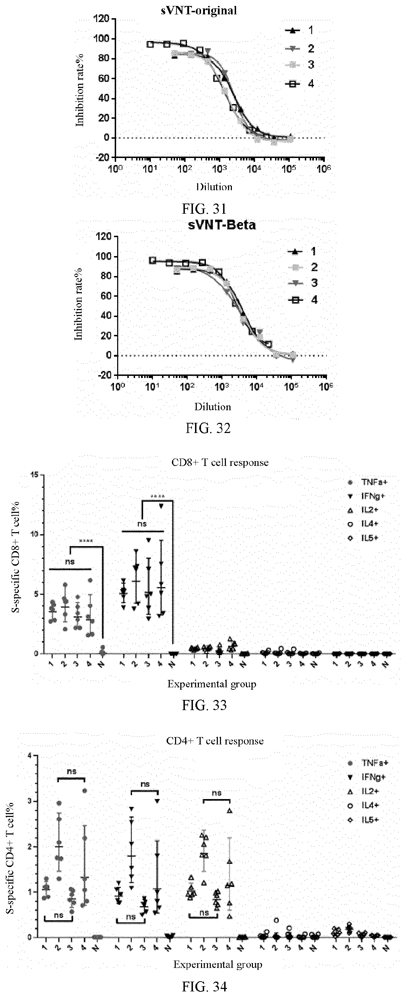

After mixing the immune serum samples on day 28 of each group, the neutralizing antibody titer was detected using a competitive ELISA method. The protein for ELISA assay plate was ACE2-Fc, while the competitive proteins were RBD protein of original strain and RBD protein of Beta variant, respectively. As shown in FIG. 31, the neutralizing antibody titers of four groups of mixed samples detected by original strain RBD protein was 2591, 2644, 1707 and 1313, respectively. As shown in FIG. 32, the neutralizing antibody titers of four groups of mixed samples detected by Beta variant RBD protein was 4144, 3542, 3534 and 2567, respectively. The trend of the detection results of the two RBD proteins was consistent, and the trend of the antibody titer detection results was also consistent. It was demonstrated that the formulation comprising the lipid compound of the present invention could elicit a higher antibody response compared to a positive control sample.

Splenocytes isolated after mouse dissection were stimulated with a full-length overlapping peptide library of S protein, and the ICS method was used to detect antigen specificity and frequency of CD4+ T cells and CD8+ T cells secreting IL-2, IFNγ, TNFα, IL-4 and IL-5, respectively. The cellular immune response of specific CD8+ T cells is shown in FIG. 33, and all experimental groups elicited Th1 class biased immune responses, with almost no CD8+ T cells secreting IL-2, IL-4 and IL-5. There was no significant difference in the ratios of IFNγ secretion specific CD8+ T cells and TNFα secretion specific CD8+ T cells in mice of each experimental group. The cellular immune response of specific CD4+ T cells is shown in FIG. 34, and all experimental groups elicited Th1 class biased immune responses, with almost no CD4+ T cells secreting IL-4 and IL-5. The ratios of IFNγ secretion specific CD8+ T cells, TNFα secretion specific CD8+ T cells and IL-2 secretion specific CD8+ T cells in groups 1-2 (samples 9 and 10) were not significantly different from those in groups 3-4 (samples 11 and 12), respectively, but the mean values were higher. It was demonstrated that the formulation comprising the lipid compound of the present invention did not elicit a specific cellular immune response inferior to the corresponding positive control sample.

## Claims

1. A lipid compound, having the following structure: 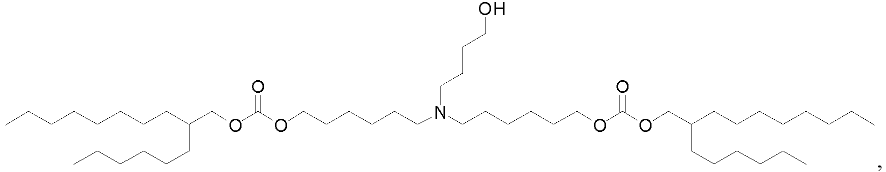 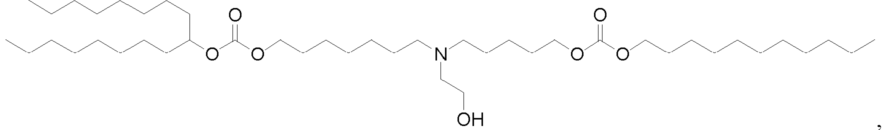 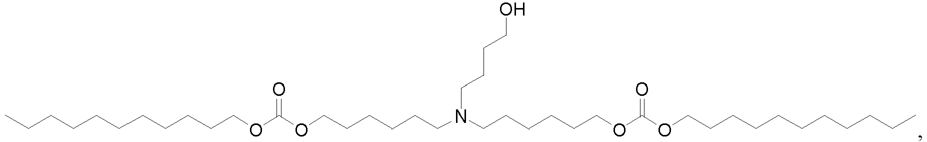 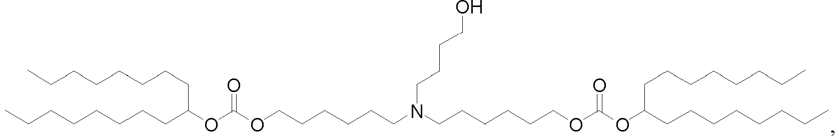 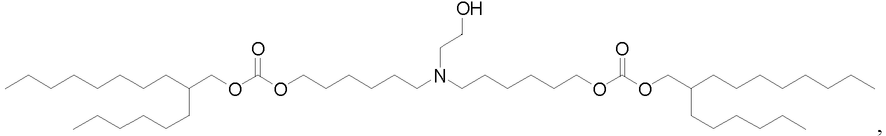 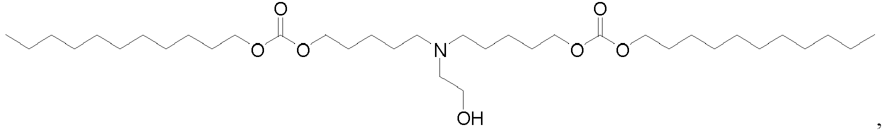 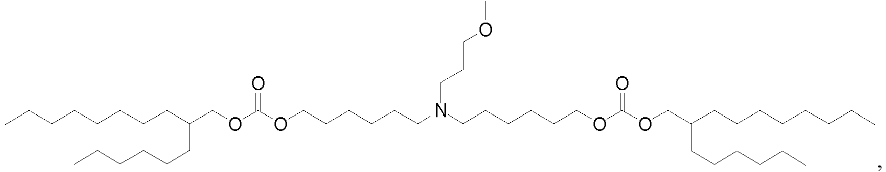 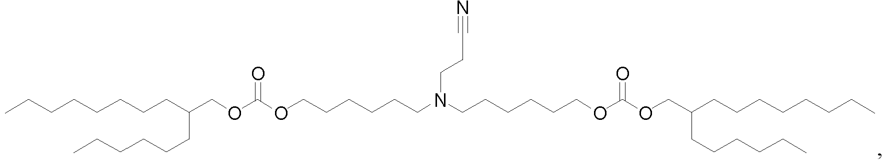 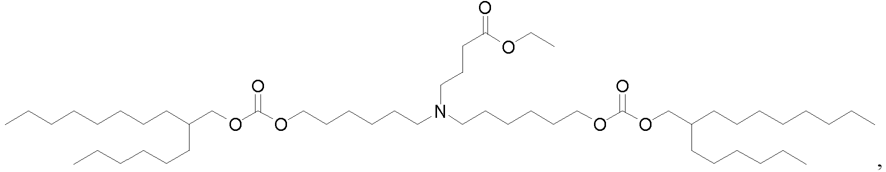 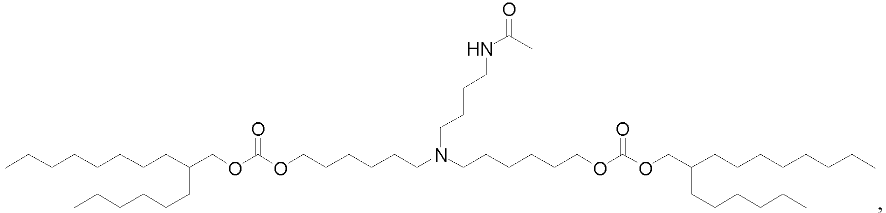 or

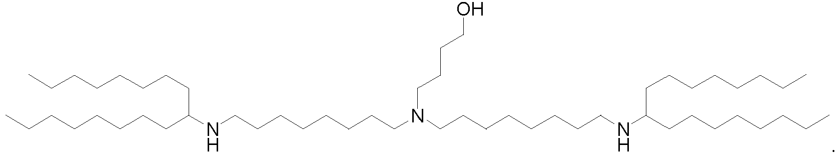

2. Use of the lipid compound according to claim 1 in the preparation of a bioactive substance delivery system.

3. The use according to claim 2, wherein the bioactive substance is DNA or RNA, and the RNA is selected from an antisense RNA, a saRNA, a mRNA, a lncRNA, a miRNA, a siRNA, a piRNA, a gRNA, and a tsRNA.

4. The use according to claim 2, wherein the bioactive substance delivery system is a mRNA vaccine.

5. The use according to claim 4, wherein the vaccine is a vaccine for preventing cancer, virus infection, bacterial infection, and fungal infection.

6. The use according to claim 5, wherein the virus is selected from: norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.

7. The use according to claim 5, wherein the virus is SARS-CoV-2.

8. A lipid nanoparticle, wherein the lipid nanoparticle comprises a bioactive substance and the lipid compound according to claim 1.

9. The lipid nanoparticle according to claim 8, wherein the lipid nanoparticle further comprises a polyethylene glycol lipid, a steroidal lipid, and a neutral lipid.

10. The lipid nanoparticle according to claim 9, wherein the polyethylene glycol lipid is selected from: 2-[(polyethylene glycol)-2000]-N,N-ditetradecylacetamide (ALC-0159), 1,2-dimyristoyl-sn-glyceromethoxypolyethylene glycol (PEG-DMG), 1,2-distearyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE), and PEG-1,2-dimyristoylacyloxypropyl-3-amine (PEG-c-DMA);
and/or, the neutral lipid is selected from: 1,2-distearoyl-sn-glycerol-3-phosphocholine (DSPC), 1,2-dipalmitoyl-sn-glycerol-3-phosphocholine (DPPC), 1,2-dioleoyl-sn-glycerol-3-phosphoethanolamine (DOPE), 1,2-dipalmitoyl-sn-glycerol-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), and **1-**palmitoyl-2-oleoyl phosphatidylethanolamine (POPE);
and/or, the steroid lipid is selected from: avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, campesterol, epicholesterol, ergosterol, fucosterol, hexahydrosterol, hydroxycholesterol, lanosterol, photosterol, fucosterol, sitostanol, sitosterol, stigmastanol, stigmasterol, cholic acid, glycocholic acid, taurocholic acid, deoxycholic acid, and lithocholic acid.

## Patentansprüche

1. Lipidverbindung mit der folgenden Struktur: 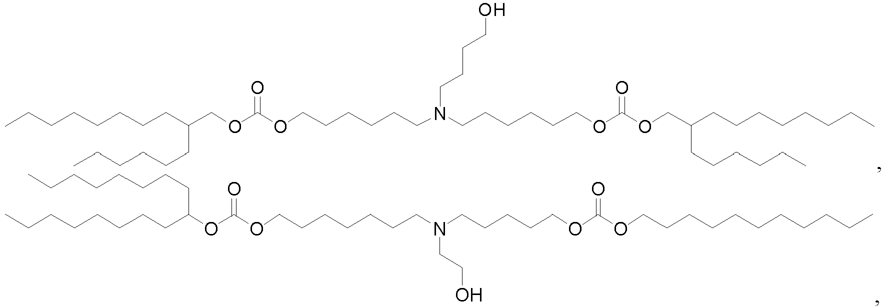 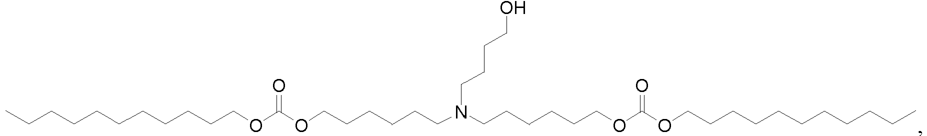 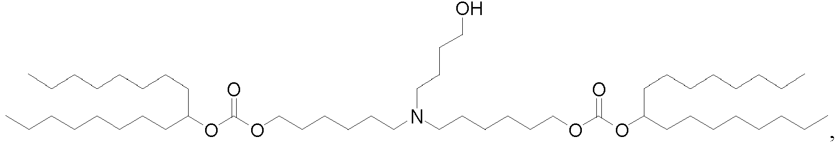 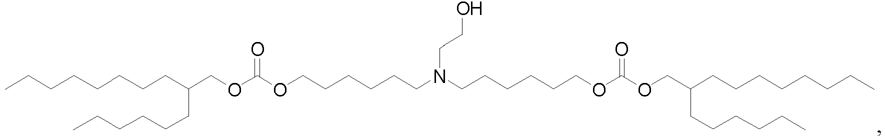 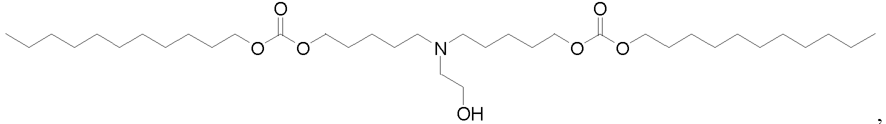 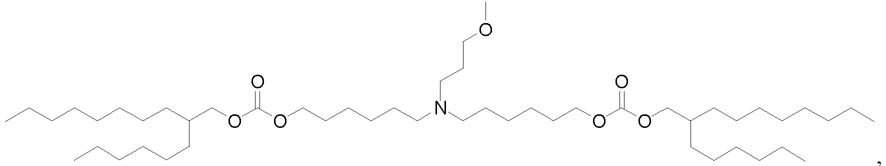 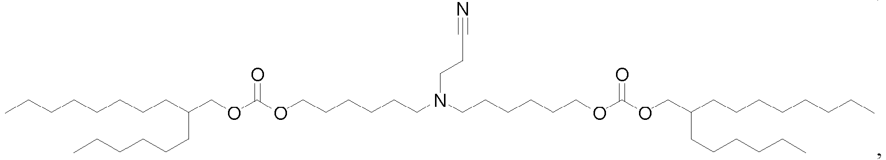 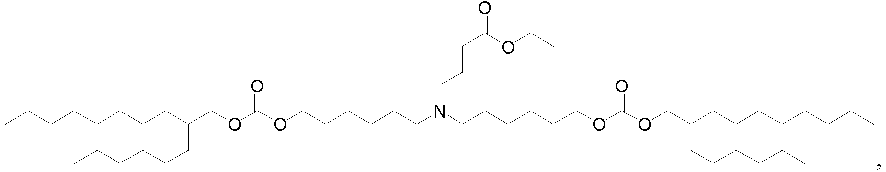 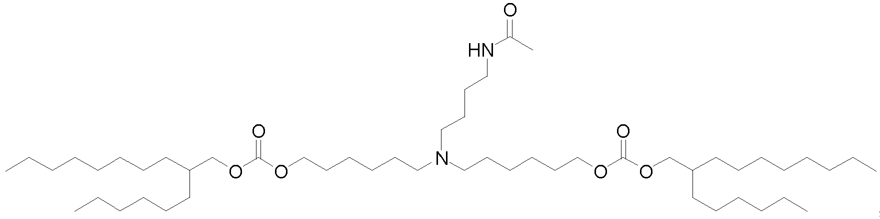 oder

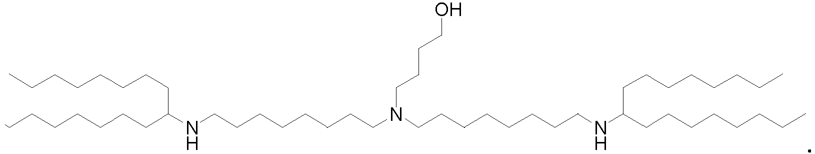

2. Verwendung der Lipidverbindung nach Anspruch 1 bei der Herstellung eines Systems zur Abgabe einer bioaktiven Substanz.

3. Verwendung nach Anspruch 2, wobei die bioaktive Substanz DNA oder RNA ist und die RNA ausgewählt ist aus einer Antisense-RNA, einer saRNA, einer mRNA, einer lncRNA, einer miRNA, einer siRNA, einer piRNA, einer gRNA und einer tsRNA.

4. Verwendung nach Anspruch 2, wobei das System zur Abgabe einer bioaktiven Substanz ein mRNA-Impfstoff ist.

5. Verwendung nach Anspruch 4, wobei der Impfstoff ein Impfstoff zum Verhindern von Krebs, Virusinfektion, bakterieller Infektion und Pilzinfektion ist.

6. Verwendung nach Anspruch 5, wobei das Virus ausgewählt ist aus: Norovirus, Ebolavirus, Coronavirus, Cytomegalovirus, Denguevirus, Zikavirus, Coxsackievirus, Enterovirus, Hepatitisvirus, Herpes-simplex-Virus, humanem Papillomavirus, Influenzavirus, Marburgvirus, Masernvirus, Poliovirus, Tollwutvirus, Rotavirus und Masernvirus.

7. Verwendung nach Anspruch 5, wobei das Virus SARS-CoV-2 ist.

8. Lipidnanopartikel, wobei der Lipidnanopartikel eine bioaktive Substanz und die Lipidverbindung nach Anspruch 1 umfasst.

9. Lipidnanopartikel nach Anspruch 8, wobei der Lipidnanopartikel ferner ein Polyethylenglycollipid, ein steroidales Lipid und ein neutrales Lipid umfasst.

10. Lipidnanopartikel nach Anspruch 9, wobei das Polyethylenglycollipid ausgewählt ist aus: 2-[(Polyethylenglycol)-2000]-N,N-ditetradecylacetamid (ALC-0159), 1,2-Dimyristoyl-snglyceromethoxypolyethylenglycol (PEG-DMG), 1,2-Distearyl-sn-glycero-3-phosphoethanolamin-N-[amino(polyethylenglycol)] (PEG-DSPE), PEG-Disterolglycerin (PEG-DSG), PEG-Dipalmitoyl, PEG-Dioleyl, PEG-Distearyl, PEG-Diacylglycerinamid (PEG-DAG), PEG-Dipalmitoylphosphatidylethanolamin (PEG-DPPE) und PEG-1,2-Dimyristoylacyloxypropyl-3-amin (PEG-c-DMA);
und/oder das neutrale Lipid ausgewählt ist aus: 1,2-Distearoyl-sn-glycerin-3-phosphocholin (DSPC), 1,2-Dipalmitoyl-sn-glycerin-3-phosphocholin (DPPC), 1,2-Dioleoyl-sn-glycerin-3-phosphoethanolamin (DOPE), 1,2-Dipalmitoyl-sn-glycerin-3-phosphoethanolamin (DPPE), 1,2-Dimyristoyl-sn-glycero-3-phosphoethanolamin (DMPE), 2-Dioleoyl-sn-glycero-3-phospho-(1'-rac-glycerol) (DOPG), Oleoylphosphatidylcholin (POPC) und 1-Palmitoyl-2-oleoylphosphatidylethanolamin (POPE);
und/oder das Steroidlipid ausgewählt ist aus: Avenasterol, β-Sitosterol, Brassicasterol, Ergocalciferol, Campesterol, Cholestanol, Cholesterin, Coprosterol, Dehydrocholesterin, Desmosterol, Dihydroergocalciferol, Dihydrocholesterin, Dihydroergosterol, Campesterol, Epicholesterin, Ergosterol, Fucosterol, Hexahydrosterol, Hydroxycholesterin, Lanosterol, Photosterol, Fucosterol, Sitostanol, Sitosterol, Stigmastanol, Stigmasterol, Cholsäure, Glycocholsäure, Taurocholsäure, Desoxycholsäure und Lithocholsäure.

## Revendications

1. Composé lipidique, présentant la structure suivante : 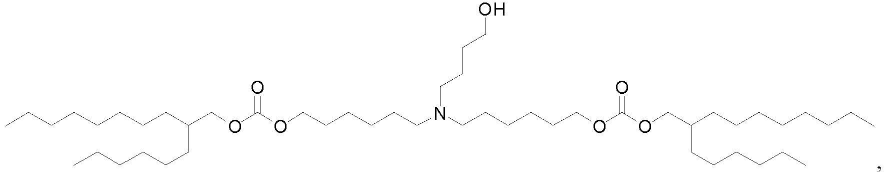 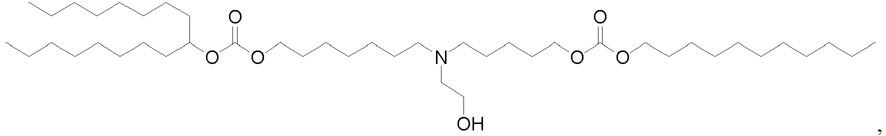 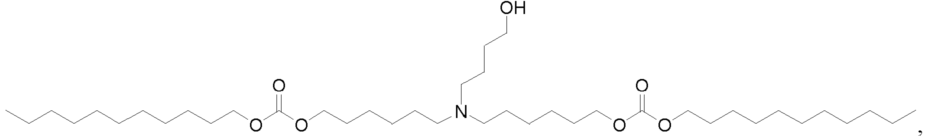 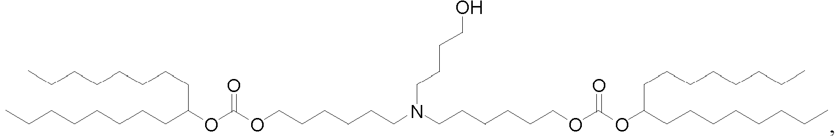 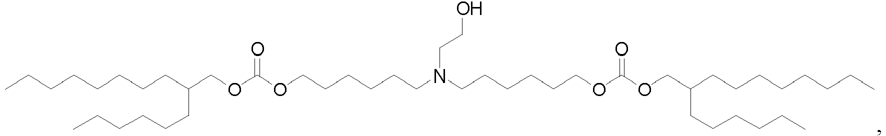 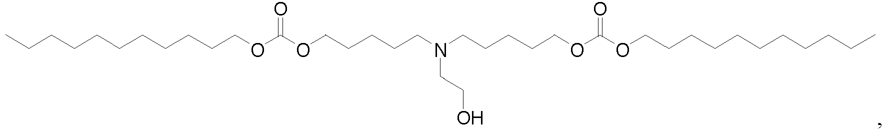 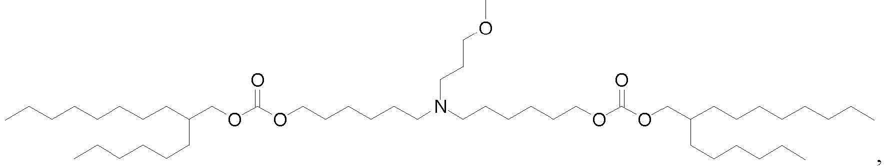 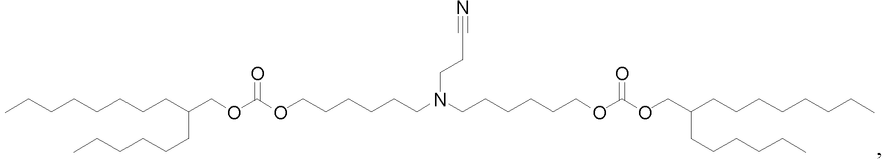 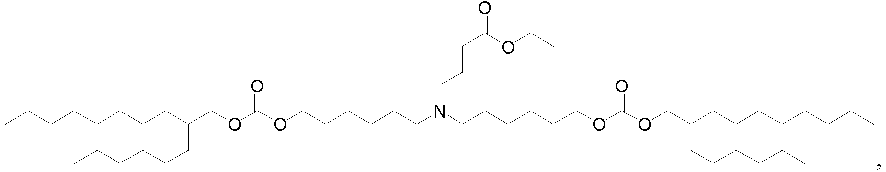 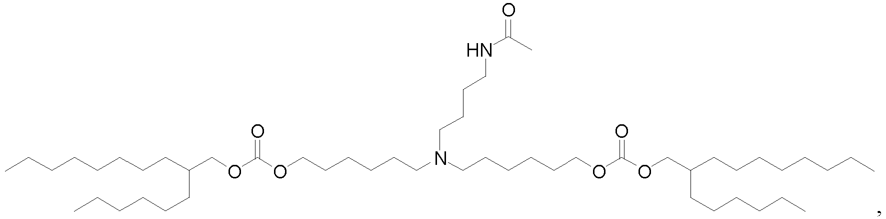 ou

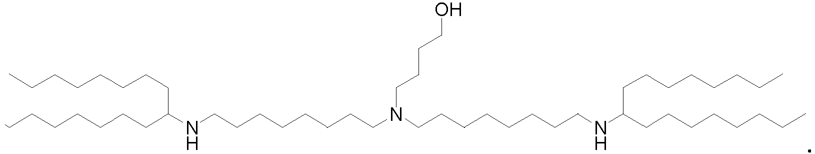

2. Utilisation du composé lipidique selon la revendication 1 dans la préparation d'un système d'administration de substance bioactive.

3. Utilisation selon la revendication 2, dans laquelle la substance bioactive est un ADN ou un ARN, et l'ARN est choisi parmi un ARN antisens, un ARNsa, un ARNm, un ARNlnc, un ARNmi, un ARNsi, un ARNpi, un ARNg et un ARNt.

4. Utilisation selon la revendication 2, dans laquelle le système d'administration de substance bioactive est un vaccin à ARNm.

5. Utilisation selon la revendication 4, dans laquelle le vaccin est un vaccin destiné à prévenir un cancer, une infection virale, une infection bactérienne et une infection fongique.

6. Utilisation selon la revendication 5, dans laquelle le virus est choisi parmi : le norovirus, le virus Ebola, le coronavirus, le cytomégalovirus, le virus de la dengue, le virus Zika, le coxsackievirus, l'entérovirus, le virus de l'hépatite, le virus de l'herpès simplex, le virus du papillome humain, le virus de la grippe, le virus de Marburg, le virus de la rougeole, le poliovirus, le virus de la rage, le rotavirus et le virus de la rougeole.

7. Utilisation selon la revendication 5, dans laquelle le virus est le SARS-CoV-2.

8. Nanoparticule lipidique, ladite nanoparticule lipidique comprenant une substance bioactive et le composé lipidique selon la revendication 1.

9. Nanoparticule lipidique selon la revendication 8, ladite nanoparticule lipidique comprenant en outre un lipide polyéthylène glycol, un lipide stéroïdien et un lipide neutre.

10. Nanoparticule lipidique selon la revendication 9, dans laquelle le lipide polyéthylène glycol est choisi parmi : le 2-[(polyéthylèneglycol)-2000]-N,N-ditétradécylacétamide (ALC-0159), le 1,2-dimyristoyl-sn-glycérométhoxypolyéthylèneglycol (PEG-DMG), le 1,2-distéaryl-sn-glycéro-3-phosphoéthanolamine-N-[amino(polyéthylèneglycol)] (PEG-DSPE), PEG-glycérol distérolé (PEG-DSG), PEG-dipalmitoyle, PEG-dioléyle, PEG-distéaryle, PEG-amide de diacylglycérol (PEG-DAG), PEG-dipalmitoylphosphatidyléthanolamine (PEG-DPPE) et PEG-1,2-dimyristoylacyloxypropyl-3-amine(PEG-c-DMA) ;
et/ou, le lipide neutre est choisi parmi : la 1,2-distéaroyl-sn-glycérol-3-phosphocholine (DSPC), la 1,2-dipalmitoyl-sn-glycérol-3-phosphocholine (DPPC), la 1,2-dioléoyl-sn-glycérol-3-phosphoéthanolamine (DOPE), la 1,2-dipalmitoyl-sn-glycérol-3-phosphoéthanolamine (DPPE), la 1,2-dimyristoyl-sn-glycéro-3-phosphoéthanolamine (DMPE), le 2-dioléoyl-sn-glycéro-3-phospho-(1'-rac-glycérol) (DOPG), l'oléoylphosphatidylcholine (POPC) et la 1-palmitoyl-2-oléoyl-phosphatidyléthanolamine (POPE) ;
et/ou, le lipide stéroïde est choisi parmi : l'avenastérol, le β-sitostérol, le brassicastérol, l'ergocalciférol, le campestérol, le cholestanol, le cholestérol, le coprosterol, le déshydrocholestérol, le desmostérol, le dihydroergocalciférol, le dihydrocholestérol, le dihydroergostérol, le campestérol, l'épicholestérol, l'ergostérol, le fucostérol, l'hexahydrostérol, l'hydroxycholestérol, le lanostérol, le photostérol, le fucostérol, le sitostanol, le sitostérol, le stigmastanol, le stigmastérol, l'acide cholique, l'acide glycocholique, l'acide taurocholique, l'acide désoxycholique et l'acide lithocholique.
